Europäisches Patentamt

European Patent Office

Office européen des brevets

(19)

(11) EP 0 381 433 B1

(12) EUROPEAN PATENT SPECIFICATION

(45) Date of publication and mention
of the grant of the patent:
**23.10.1996 Bulletin 1996/43**

(51) Int Cl.6: **C12N 15/16**, C12P 21/02,
C12N 15/62, C12N 1/20
// (C12N1/20, C12R1:19)

(21) Application number: **90300937.1**

(22) Date of filing: **30.01.1990**

(54) **A method for the production of glucagon**

Methode zur Produktion von Glukagon

Méthode de production de glucagon

(84) Designated Contracting States:
**AT BE CH DE DK ES FR GB GR IT LI LU NL SE**

(30) Priority: **01.02.1989 JP 24158/89**

(43) Date of publication of application:
**08.08.1990 Bulletin 1990/32**

(73) Proprietor: **SHIONOGI SEIYAKU KABUSHIKI
KAISHA trading under the name of
SHIONOGI & CO. LTD.
Osaka 541 (JP)**

(72) Inventors:
• **Ishizaki, Jun
Takarazuka-shi, Hyogo-ken (JP)**
• **Tamaki, Mikio
Nara-shi, Nara-ken (JP)**
• **Shin, Masaru
Kobe-shi, Hyogo-ken (JP)**
• **Teraoka, Hiroshi
Sakai-shi, Osaka (JP)**
• **Yoshida, Nobuo
Nishinomiya-shi, Hyogo-ken (JP)**
• **Tsuzuki, Hiroshige
Tsuzuki-gun, Kyoto (JP)**

(74) Representative: **Nash, David Allan et al
Haseltine Lake & Co.
28 Southampton Buildings
Chancery Lane
London WC2A 1AT (GB)**

(56) References cited:
EP-A- 0 189 998          EP-A- 0 234 888

• **E.L. WINNACKER et al.: "From genes to clones",
chapter 7.3, 1987, pages 276- 279, VCH
Verlagsgesellschaft**
• **THE JOURNAL OF BIOCHEMISTRY, vol. 102, no.
3, September 1987, pages 607-612, Tokyo, JP; N.
KIKUCHI et al.**

**Description**

The present invention relates to an efficient method for the production of glucagon by preparation of a glucagon fusion protein followed by enzymatic treatment and recovery of glucagon, a DNA sequence encoding glucagon used in the said producing method, an expression vector comprising the said DNA sequence, and a transformant carrying this expression vector.

Glucagon is a peptide hormone secreted by the A cells of the pancreatic islets, and along with insulin secreted by the B cells of the pancreatic islets plays an important role for carbohydrate metabolism. Glucagon is also known as hyperglycemic-glycogenolytic factor (HGF); whereas insulin enhances glucose metabolism and decreases blood glucose levels, glucagon acts to release glucose into the blood and thus elevate blood glucose concentrations. By virtue of these properties, glucagon is useful in testing for growth hormone secretion, diagnosis of insulinoma, glycogen storage testing, emergency treatment of hypoglycemia, pretreatment in radiographic and endoscopic examination of the digestive tract and other clinical applications.

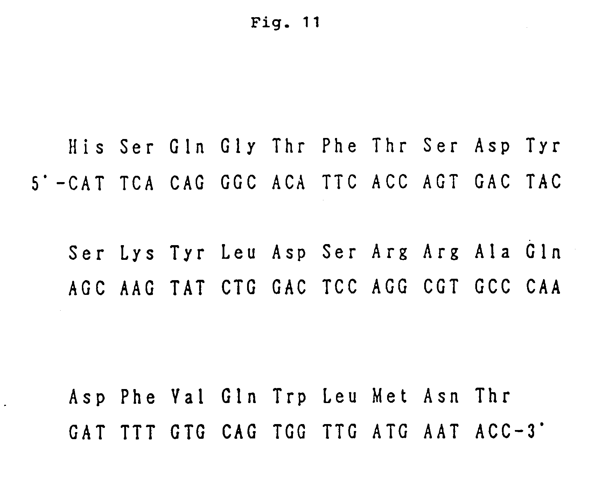

The amino acid sequence of glucagon is already known; the molecule is a single-chain peptide consisting of 29 amino acid residues beginning with histidine at the amino terminus and ending with threonine at the carboxyl terminus (see Figure 11). The amino acid sequences of human, porcine and bovine glucagons are identical, and therefore glucagon is industrially produced by isolation and purification from the pancreas of cattle or swine. Glucagon products prepared in this manner are commercially available. Furthermore, the human DNA sequence encoding glucagon has been elucidated by James W. White and Grady F. Saunders (Nucleic Acids Research, 14, 4719-4730 (1986)) (see Figure 11). Therefore, attention has been directed toward the possibility of economical mass production of glucagon using recombinant DNA techniques.

However, as mentioned above, glucagon is a short-chain peptide composed of 29 amino acid residues and therefore has a low molecular weight. When such proteins with low molecular weight are directly expressed in host cells by recombinant DNA techniques, they may be degraded by the proteolytic enzymes (proteases) present in the host cells. Thus, the expression of such low molecular weight proteins in combination with other proteins in the form of more stable fusion proteins of an appropriate size is advantageous. The target protein can then be isolated by chemical or enzymatic treatment of these fusion proteins. For example, Japanese Laid-Open Patent Publication No. 63-284196 discloses a method for recovering target proteins by chemical treatment of fusion proteins. However, chemical treatment is often accompanied with undesirable side reactions. An example of a method for the isolation of target proteins by enzymatic treatment of fusion proteins is disclosed in Japanese Laid-Open Patent Publication No. 62-246600. This publication discloses the recovery of epidermal growth factor (EGF) by treatment of a fusion protein containing EGF with a glutamic acid-specific protease. However, the EGF molecule is known to contain four glutamic acid residues, therefore the recover of EGF in complete form using this enzyme is difficult, and the yield is low. Up until the present time, no efficient method for the isolation of glucagon from fusion proteins by this type of enzymatic treatment has been known.

EP-A-0189998 describes a process for preparing glucagon in a fusion protein wherein glucagon is linked to a leader peptide. In particular, EP-A-0189998 teaches the production of glucagon in S. cerevisiae.

EP-A-0234888 describes a process for producing a fusion protein containing Epidermal Growth Factor attached to a leader peptide.

N. Kikuchi et al., J. Biochem., Vol. 102, pp 607-612, 1987 describes the use of γ interferon as a leader peptide.

According to the present invention there is provided a method for producing glucagon, which overcomes the above-discussed and numerous other disadvantages and deficiencies of the prior art, comprising the steps of:

preparing a fusion protein shown by the following formula:

X-Glu-Glucagon

wherein X is a leader sequence consisting of amino acid sequence from Gln in the 4 position to Phe in the 95 position of human interferon-γ or the amino acid sequence from Gln in the 4 position to Ser in the 135 position of human interferon-γ, Glu is a glutamic acid residue, and Glucagon represents the amino acid sequence of glucagon, by cultivating transformants obtained by introducing an expression vector for expressing said fusion protein into E. Coli and allowing the E. Coli to express said fusion protein; and

treating said fusion protein with an enzyme that specifically hydrolyzes the peptide bonds at the carboxyl termini of the glutamic acid residues, resulting in cleavage of said fusion protein.

In a preferred embodiment of the invention, said amino acid sequence of glucagon is shown by the followina formula:

```
His Ser Gln Gly Thr Phe Thr Ser Asp Tyr
Ser Lys Tyr Leu Asp Ser Arg Arg Ala Gln
Asp Phe Val Gln Trp Leu Met Asn Thr          (II)
```

In a preferred embodiment, the glucagon is recovered by the following procedure which comprises the steps of:

(a) solubilizing said fusion protein with an appropriate denaturant or surfactant,

(b) lowering the concentration of said denaturant or surfactant,

(c) adding the enzyme to the mixture obtained in step (b), thereby obtaining a reaction mixture containing soluble intermediates arising from the partial decomposition of said fusion protein,

(d) desalinizing said reaction mixture to obtain a fraction containing said intermediates and said enzyme, and containing neither said denaturant nor said surfactant,

(e) allowing the reaction to further proceed by means of the enzyme contained in said fraction to release the glucagon, and

(f) isolating the glucagon released.

The enzyme employed to hydrolyze the peptide bonds is preferably protease V8 derived from Staphylococcus Aureus.

An expression vector of the present invention possesses a DNA sequence encoding the above-mentioned fusion protein, and efficiently expresses the said fusion protein in Escherichia coli host cells.

The transformants of the present invention are obtained by introducing the above-mentioned expression vector into a cell of Escherichia coli.

A purified glucagon made in accordance with this invention is of 99 percent purity or more, said purity being determined by high performance liquid chromatography.

In accordance with another aspect of the invention, there is provided a method for producing glucagon, comprising:

preparing a fusion protein by cultivating transformants obtained by introducing an expression vector for expressing a fusion protein shown by formula (I) into Escherichia coli and allowing the Escherichia coli to express the protein of formula (I):

$$X\text{-Glu-Glucagon} \qquad (I)$$

wherein X is a leader sequence consisting of amino acid sequence from Gln in the 4 position to Phe in the 95 position of human interferon-$\gamma$ or the amino acid sequence from Gln in the 4 position to Ser in the 135 position of human interferon-$\gamma$, Glu is a glutamic acid residue, and Glucagon represents the amino acid sequence of glucagon, wherein said Glucagon is encoded by a DNA sequence shown by the following formula:

```
5'-CACTCTCAGGGTACCTTCACTTCCGACTACTCTAAA

  TACCTGGACTCCCGTCGTGCTCAGGACTTCGTTCAG

  TGGCTGATGAACACC-3'                    (III)
```

treating said fusion protein with an enzyme that specifically hydrolyzes the peptide bonds at the carboxyl termini of the glutamic acid residues, resulting in cleavage of said fusion protein.

Thus, the invention described herein makes possible the objectives of:

(1) providing a method for the production of glucagon in which a fusion protein containing glucagon is prepared and the glucagon is recovered by an enzymatic treatment with high efficiency;

(2) providing an economical method for the production of glucagon which is identical with natural glucagon by the above-mentioned method, using small amount of enzymes;

(3) providing an expression vector possessing a DNA sequence encoding the aforementioned fusion protein, and permitting the efficient expression of the said DNA sequence in Escherichia coli, as well as a transformant in which the said expression vector is introduced; and

(4) providing a purified glucagon of 99 percent purity or more.

For a better understanding of the invention and to show how the same can be carried into effect, reference will be made, by way of example only, to the following drawings, in which:-

Figure 1 shows the DNA sequence encoding human glucagon.

Figure 2a is a schematic diagram showing the construction of the plasmid pGA1 used in the construction of the expression vector Trp pAT huIFN γ/glucagon of the present invention.

Figure 2b is a schematic diagram showing the construction of the plasmid Trp pAT hUIFN γ/Kallikrein used in the construction of the expression vector Trp pAT huIFN γ/glucagon of the present invention.

Figure 2c is a schematic diagram showing the construction of the expression vector Trp pAT huIFN γ/glucagon of the present invention.

Figure 3 shows the base sequence of the DNA encoding glucagon contained in the expression vector Trp pAT hUIFN γ/glucagon as well as the adjacent regions on the upstream and downstream sides of the DNA.

Figure 4a is a schematic diagram showing the construction of the plasmid Trp pAT ΔCys huIFNγ used in the construction of the expression vector Trp pAT ΔCys huIFN γ/glucagon.

Figure 4b is a schematic diagram showing the construction of the expression vector Trp pAT ΔCys huIFN γ/glucagon.

Figure 5 is a schematic diagram showing the construction of the expression vector Trp pAT ΔCys short huIFN γ/glucagon of the present invention.

Figure 6 is a schematic diagram showing the construction of the expression vector Trp pAT huIFN α 80A2/glucagon.

Figure 7a is a schematic diagram showing the synthesis by the PCR method of the DNA fragment containing the base sequence encoding human IFN α 80A2.

Figure 7b is a schematic diagram showing the amplification by the PCR method of the gene sequence encoding glucagon as well as the upstream and downstream DNA segments in the construction of the expression vector Trp pAT ΔCySII hUIFN γ/glucagon.

Figure 7c is a schematic diagram showing the construction of the expression vector Trp pAT Δ CysII huIFN γ/glucagon.

Figure 8 shows the DNA sequence of human IFN α 80A2 and the corresponding amino acid sequence.

Figures 9a and 9b are charts showing the results of reverse phase HPLC and ion exchange HPLC analysis of the glucagon obtained by the recombinant DNA technique of the present invention.

Figures 10a and 10b are charts showing the results of reverse phase HPLC of the glucagon obtained using recombinant DNA techniques by the method of the present invention.

Figure 10c is a chart showing the result of reverse phase HPLC of the glucagon commercially available natural glucagon.

Figure 11 shows the amino acid sequence of human glucagon and the human DNA sequence encoding the said glucagon.

In the fusion protein X-Glu-Glucagon prepared in the method of the present invention, an amino acid sequence from Gln in the 4 position to Phe in the 95 position of human interferon-γ or from Gln in the 4 position to Ser in the 135 position of human interferon-γ displaying a high level of expression is selected as the leader sequence denoted by X. The leader sequence X is such that when the base sequence encoding X is introduced into E. coli, a suitable host, to induce expression, the said fusion protein accounts for 20% or more of total protein production, and moreover, such that the said fusion protein forms granules (i.e., inclusion bodies) in the host cell. An expression vector possessing a DNA sequence encoding a polypeptide of a form such that the above-mentioned sequences are linked with glucagon via a glutamic acid residue is constructed, and E. coli is transformed with this expression vector, thereby permitting the high efficiency expression of the said polypeptide in the form of stable fusion protein granules.

In the following, the producing method of glucagon of the present invention will be described in accordance with the order of the producing process. The recombinant DNA techniques employed in this process can be implemented, for example, by the procedures described in "Molecular Cloning" (Cold Spring Harbor Laboratory, New York, 1982).

Design and chemical synthesis of DNA sequences encoding glucagon

The amino acid sequences of human, porcine and bovine glucagons are identical, being represented by the following formula:

```
His Ser Gln Gly Thr Phe Thr Ser Asp Tyr
Ser Lys Tyr Leu Asp Ser Arg Arg Ala Gln
Asp Phe Val Gln Trp Leu Met Asn Thr      (II)
```

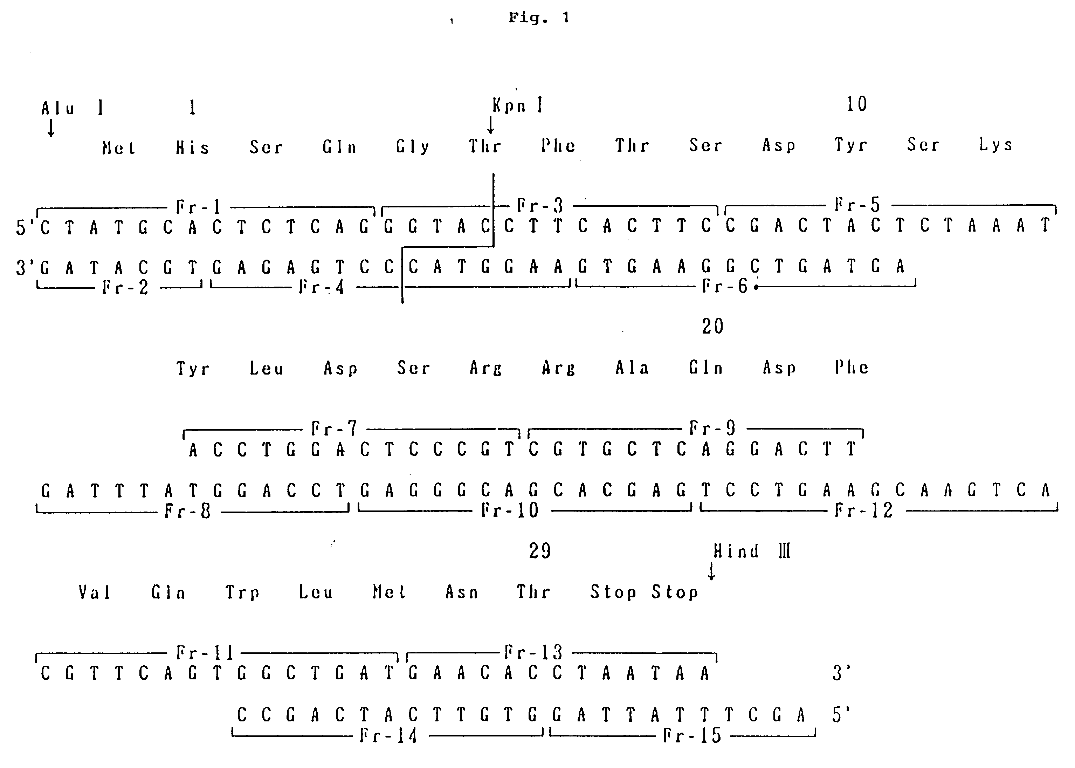

The DNA sequence encoding human glucagon has, for example, been elucidated by James W. White and Grady F. Saunders (supra). The amino acid sequence of human glucagon and the human DNA sequence encoding the said glucagon are shown in Figure 11. Although this DNA sequence can be used in the present invention, the use of DNA sequences preferentially employing codons which are utilized with high frequency in E. coli is particularly advantageous. Accordingly, the inventors have designed and synthesized a DNA sequence containing, as far as possible, codons used with high frequency in E. coli. This DNA sequence is shown in Figure 1.

The DNA sequence encoding glucagon is comparatively short, and can therefore be obtained, for example, by chemical synthesis. This synthetic DNA can be prepared, for example, by first synthesizing the 15 fragments indicated in Figure 1 (Fr 1-15) with an automatic nucleic acid synthesizer, purifying these fragments by chromatography such as high performance liquid chromatography (HPLC), treating each fragment with kinase, and ligating the fragments with DNA ligase by conventional methods. The DNA obtained by ligation of the said fragments is then isolated by conventional methods such as phenol extraction, ethanol precipitation, agarose gel electrophoresis, etc. DNA fragments can be recovered from the agarose gel by elution in accordance with the procedure described in Nucl. Acids Res., 8, 253-264 (1980).

Then, recombinant plasmids (i.e., expression vectors) are constructed using these glucagon-encoding DNA sequences.

Construction of expression vectors

The construction of the expression vectors of the present description i.e., (a) Trp pAT huIFN γ/glucagon, (b) Trp pAT ΔCys huIFN γ/glucagon, (c) Trp pAT ΔCys short huIFN γ/glucagon, (d) Trp pAT hUIFN α 80A2/glucagon comparative and (e) Trp pAT ΔCysII hUIFN γ/glucagon, will be described below.

(a) Construction of Trp pAT hUIFN γ/glucagon

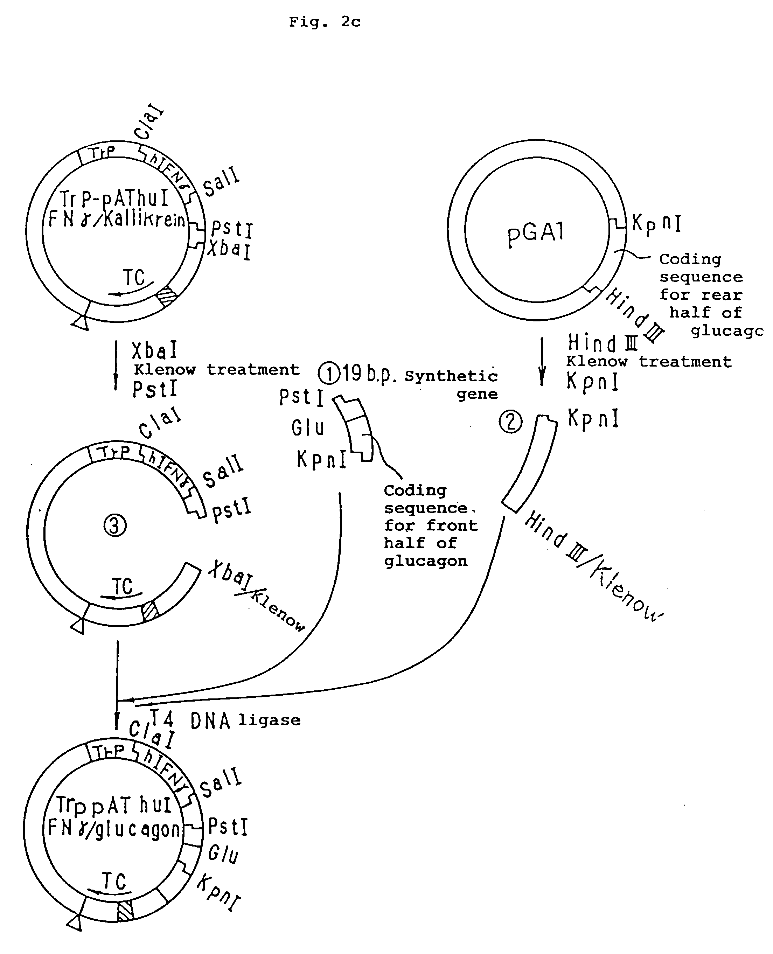

As shown by Figure 2c, the expression vector Trp pAT huIFN γ/glucagon is constructed using the following three components:

(1) a 19 bp fragment containing a base sequence encoding the front half of the amino acid sequence of glucagon;

(2) a fragment containing a base sequence encoding the rear half of the amino acid sequence of glucagon; and

(3) the Trp pAT huIFNγ/Kallikrein vector.

The structure of the DNA fragment (1) is shown by the following formula:

```
        1   2   3   4
     GluHisSerGlnGly(Thr)
    GCGAACACTCTCAGGGTAC
ACGTCGCTTGTGAGAGTCC
      PstI              KpnI                (IV)
```

This fragment is composed of a DNA sequence encoding the N-terminal amino acid sequence of the glucagon comprising histidine (His), serine (Ser), glutamine (Gln), glycine (Gly), plus a portion of the threonine (Thr), to the 5′ end of which is attached the glutamic acid (Glu) codon GAA. Also, the fragment has a cleavage site for PstI at the 5′-terminus, and having a cleavage site for KpnI at the 3′-terminus. This fragment is obtained by chemically synthesizing and then annealing the two types of single-stranded 19-base oligonucleotides which constitute the fragment.

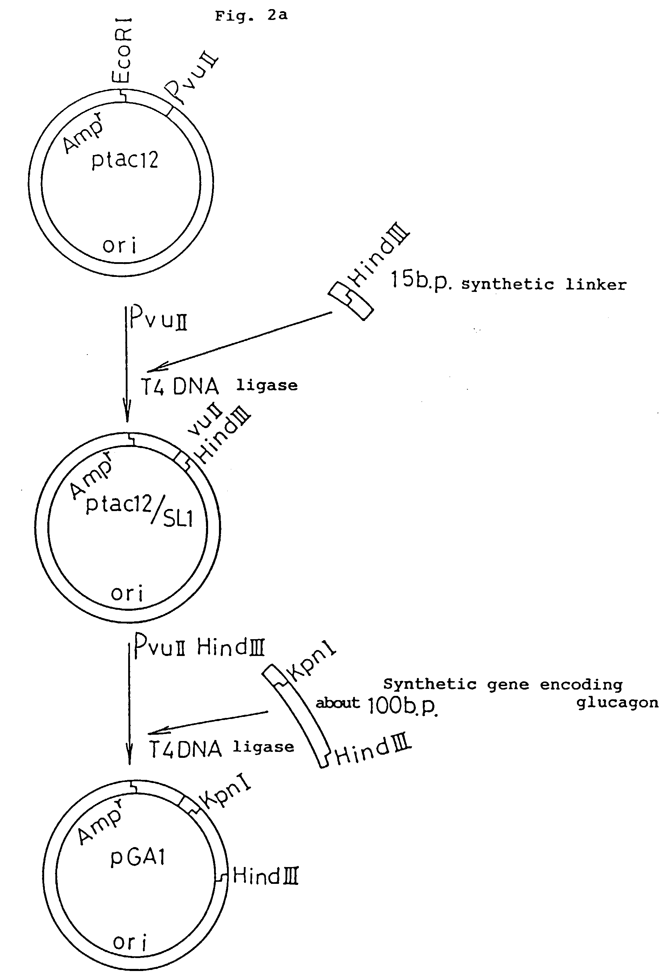

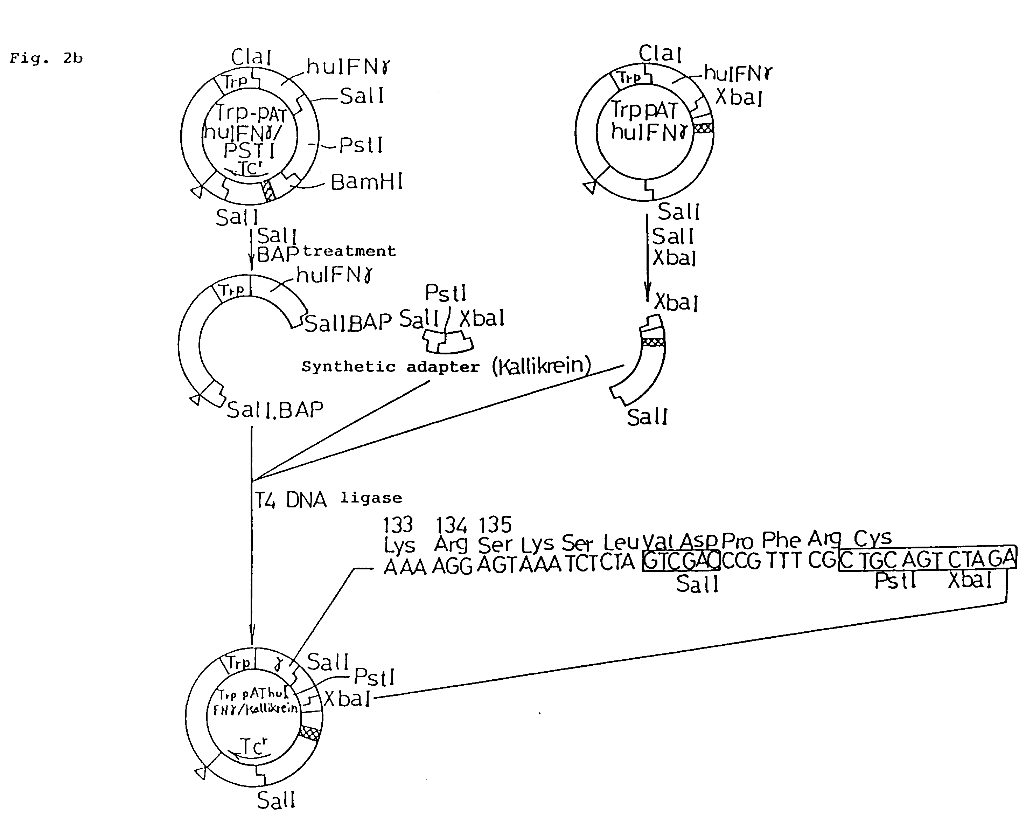

The fragment (2), is obtained by restriction enzyme cleavage of the cloning vector pGA1 constructed from ptac12 (Amman et al., Gene, 25, 167, 1983) as prototype in the manner shown in Figure 2a. Specifically, pGA1 is constructed from ptac12 by the following procedure. First, the synthetic linker (V) shown by the following formula is inserted into the PvuII cleavage site in ptac12.

```
5'- CTGAAGCTTGGATCC -3'
3'- GACTTCGAACCTAGG -5'
          HindIII                              (V)
```

The plasmid so obtained (i.e., ptac12/SL1; possessing a HindIII cleavage site downstream from the PvuII cleavage site) is then cleaved with both PvuII and HindIII, and then the approximately 100 bp synthetic gene shown in Figure 1 is inserted. Then, as shown in Figure 2c, the plasmid pGA1 obtained in this manner is cleaved with HindIII, the ends are blunted by treatment with Klenow fragment, and the DNA fragment so obtained is then cleaved with KpnI, resulting in a 90 bp KpnI-HindIII/Klenow fragment.

The Trp pAT huIFNγ/Kallikrein vector (3) is obtained as follows. First, as shown in Figure 2b, the expression plasmid for PSTI fused with human interferon-γ (i.e., Trp pAT huIFNγ/PSTI, J. Biochem., 102, 607-612, 1987) is treated with SalI and BAP, and the larger of the two resulting fragments (i.e., the SalI-SalI fragment) is recovered. Separately, the Trp pAT huIFNγ plasmid is digested with SalI and XbaI, thereby obtaining a SalI-XbaI fragment. Next, the following oligonucleotide adapter VI (containing a DNA sequence encoding the recognition site (Phe-Arg) of human plasma kallikrein) is synthesized.

```
SalI Asp Pro Phe Arg    PstI   XbaI
   TC GAC CCG TTT CGC  TGC AGT
     G GGC AAA GCG ACG TCA GATC        (VI)
```

The aforementioned SalI-SalI fragment, SalI-XbaI fragment and synthetic oligonucleotide adapter VI are then ligated with T4 DNA ligase to obtain the plasmid vector Trp pAT huIFNγ/Kallikrein. This vector Trp pAT huIFNγ/Kallikrein possesses a sequence encoding the segment of human interferon-γ up to the serine residue at the 135 position under the control of the tryptophan promoter, followed by the sequence shown in the lower portion of Figure 2b, at the downstream area.

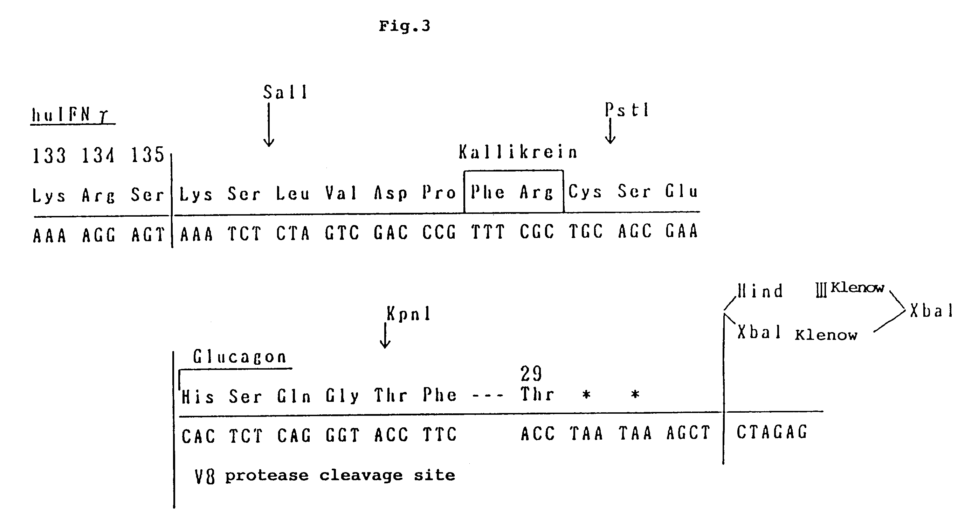

The aforementioned Trp pAT huIFNγ/Kallikrein vector (3) is treated with XbaI, Klenow fragment and PstI, and the larger of the fragments so obtained is ligated to the aforementioned fragments (1) and (2) using T4 DNA ligase in the conventional manner, thereby obtaining the expression vector Trp pAT huIFNγ/glucagon (Figure 2c). The base sequences adjacent to the 5′ and 3′ termini of the DNA encoding glucagon in this expression vector are shown in Figure 3.

(b) Construction of Trp pAT ΔCys huIFNγ/glucagon

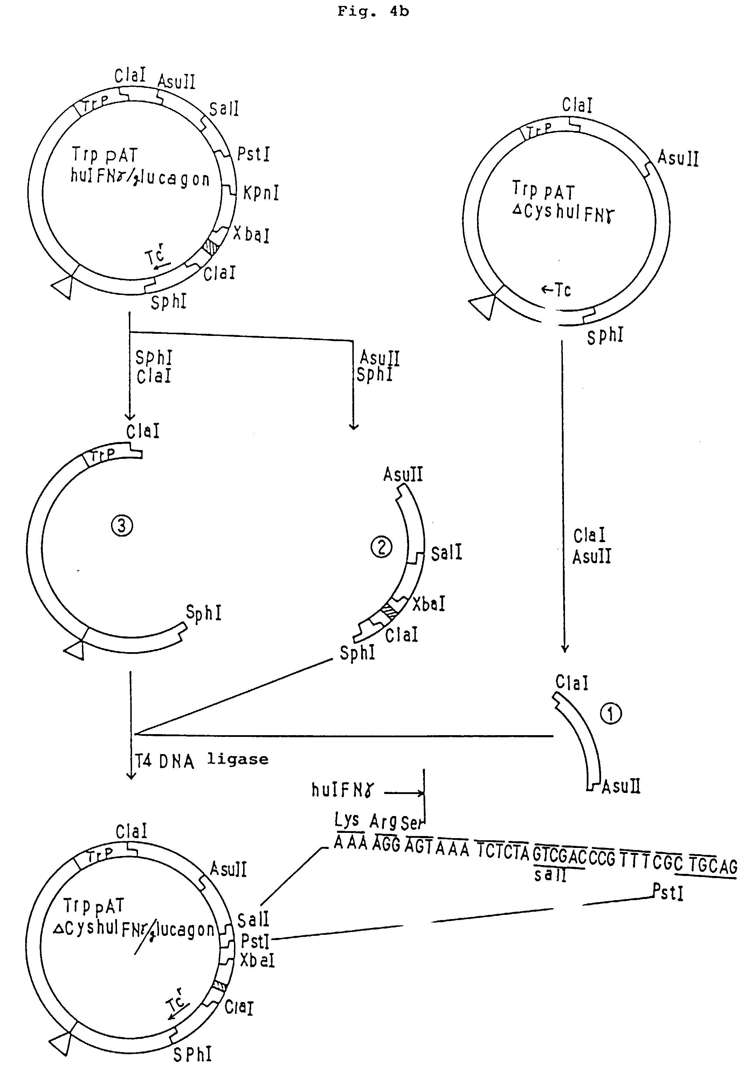

As shown by Figure 4b, the expression vector Trp pAT ΔCys huIFNγ/glucagon is constructed using the following three components:

(1) a fragment containing a base sequence encoding the N-terminal amino acid sequence (front half) of ΔCys huIFNγ;

(2) a fragment containing a base sequence encoding the C-terminal amino acid sequence (rear half) of ΔCys huIFNγ and a sequence encoding glucagon; and

(3) a Trp pAT vector.

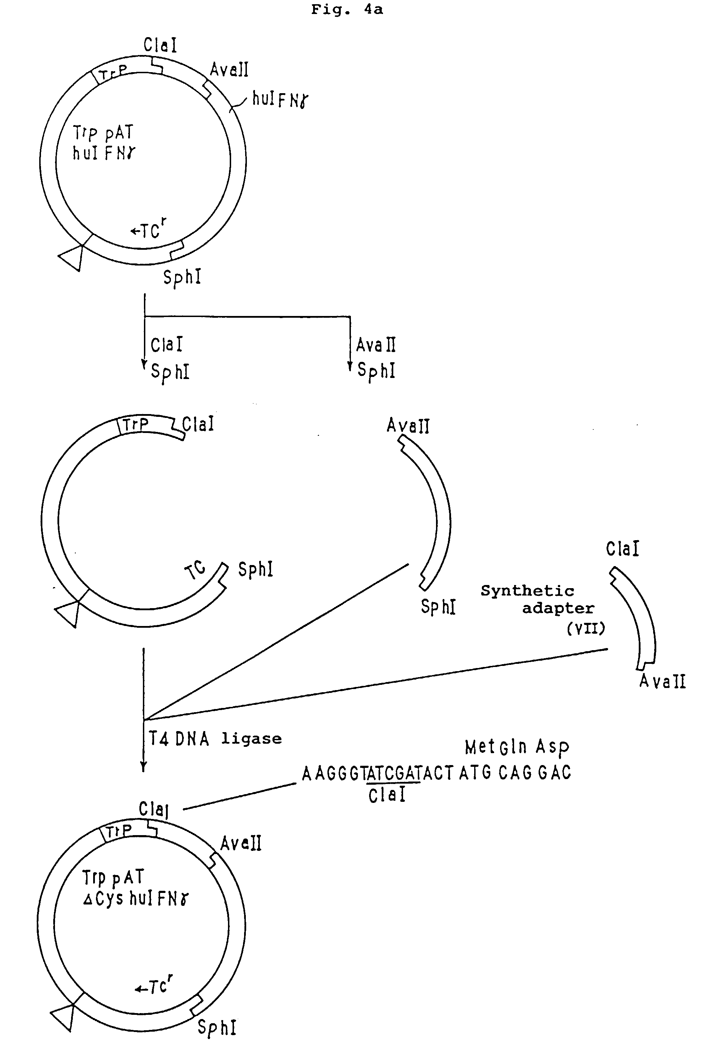

As shown in Figure 4b, the DNA fragment (1) is obtained from the huIFNγ expression plasmid Trp pAT ΔCys huIFNγ (see Figure 4a) constructed from Trp pAT huIFNγ as prototype (Kiso to Rinsho, Vol. 20, pp. 4029-4034 (1986), Japan). The construction of Trp pAT ΔCys huIFNγ from Trp pAT huIFNγ is as follows. First, Trp pAT huIFNγ is digested with ClaI, AvaII and SphI, thus obtaining a ClaI-SphI fragment and an AvaII-SphI fragment. Next, the following oligonucleotide (VII) is synthesized.


```
                    MetGln(Asp)
         5'-CGATACTATGCAG      -3'
         3'-   TATGATACGTCCTG -5'
            ClaI            AvaII                          (VII)
```


This synthetic oligonucleotide adapter (VII) encodes the first part of ΔCys huIFNγ, beginning from the glutamine (Gln) and aspartic acid (Asp) residues at the amino terminus of the ΔCys huIFNγ. The oligonucleotide (VII) is obtained by synthesizing the two indicated single-strand oligonucleotides, with respective lengths of 13 and 14 bases, and annealing the said two single-strand oligonucleotides.

The plasmid vector Trp pAT ΔCys huIFNγ is then constructed by ligating the aforementioned ClaI-SphI fragment, AvaII-SphI fragment and synthetic adapter (VII) with T4 DNA ligase. Then, this Trp pAT ΔCys huIFNγ vector is digested with the restriction enzymes ClaI and AsuII, thereby obtaining a 275 bp fragment. This ClaI-AsuII fragment encodes the portion of human interferon-γ beginning from the glutamine residue at the 4th position up to a point corresponding to an AsuII cleavage site in the base sequence.

The DNA fragment (2) is the smaller of the fragments resulting from cleavage with AsuII and SphI of the expression vector Trp pAT huIFNγ/glucagon (Figure 2c) obtained in the foregoing item (a). This AsuII-SphI fragment contains a base sequence encoding the rear half of the amino acid sequence of human interferon-γ, beginning from the point corresponding to the AsuII cleavage site within the base sequence encoding human interferon-γ, and also, downstream to this, a base sequence encoding the amino acid sequence of glucagon.

The DNA fragment (3) is the larger of the fragments resulting from cleavage with ClaI and SphI of the aforementioned expression plasmid Trp pAT huIFNγ/glucagon shown in Figure 4b. This ClaI-SphI fragment contains the base sequence of the tryptophan promoter.

The expression vector Trp pAT ΔCys huIFNγ/glucagon is obtained by ligating the aforementioned ClaI-AsuII fragment (1), AsuII-SphI fragment (2) and ClaI-SphI fragment (3) in the conventional manner using T4 DNA ligase (see Figure 4b).

This vector Trp pAT ΔCys huIFNγ/glucagon possesses a sequence encoding the segment of human interferon-γ from the Gln residue at the 4th position to the Ser residue at the 135th position under the control of the tryptophan promoter, followed by the sequence shown in the lower portion of Figure 4b, at the downstream area. The base sequences adjacent to the 5′ and 3′ termini of the DNA encoding glucagon in this expression vector are the same as those in Trp pAT huIFNγ/glucagon, which are shown in Figure 3.

(c) Construction of Trp pAT ΔCys short huIFNγ/glucagon

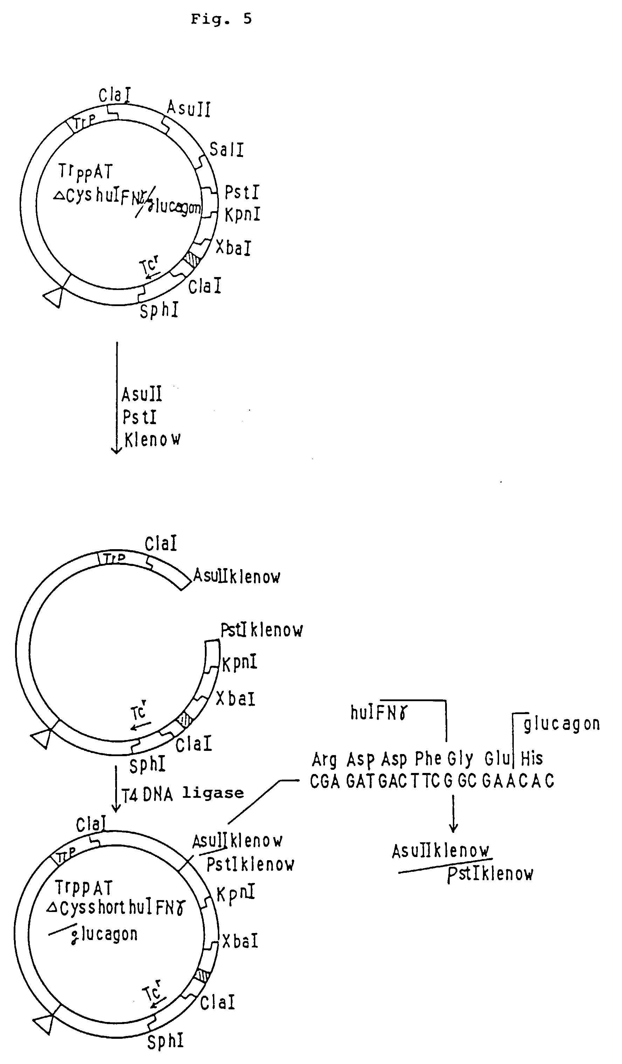

As shown by Figure 5, the expression vector Trp pAT ΔCys short huIFNγ/glucagon of the present invention is constructed from the expression vector Trp pAT ΔCys huIFNγ/glucagon obtained in item (b) (Figure 4b).

Specifically, Trp pAT ΔCys short huIFNγ/glucagon is obtained by the following procedure. First, the vector Trp pAT ΔCys huIFNγ/glucagon is cleaved with the restriction enzymes AsuII and PstI, and blunted with Klenow fragment. The larger of the fragments so obtained (AsuII/Klenow-PstI/Klenow fragment) is then ligated in the conventional manner with T4 DNA ligase, thereby obtaining the vector Trp pAT ΔCys short huIFNγ/glucagon.

This vector Trp pAT ΔCys short huIFNγ/glucagon possesses a gene sequence encoding the segment of human interferon-γ from the glutamine residue at the 4th position up to the phenylalanine (Phe) residue at the 95th position of human interferon-γ under the control of the tryptophan promoter, and on the downstream side, following an interposed sequence encoding glycine and glutamic acid residues, contains a base sequence encoding the amino acid sequence of glucagon. The base sequence adjacent to the 5' terminus of the DNA encoding glucagon in this expression vector is shown in the lower right-hand portion of Figure 5.

(d) Construction of Trp pAT hUIFN α 80A2/glucagon

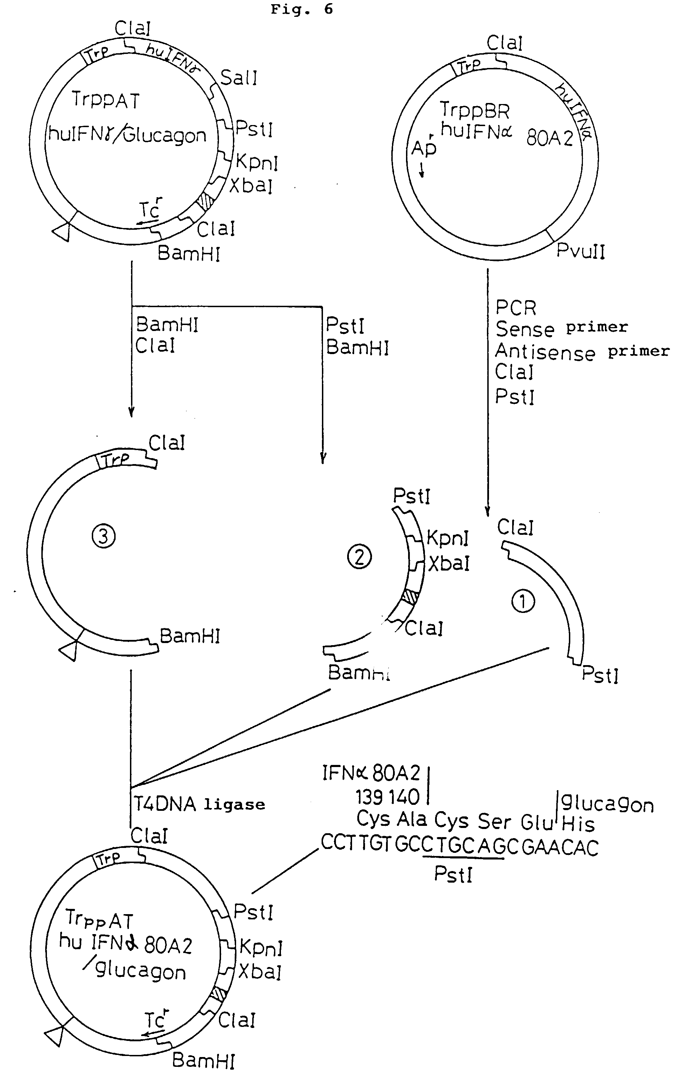

As shown in Figure 6, the expression vector Trp pAT huIFN α 80A2/glucagon is constructed using the following three components:

(1) a fragment containing a base sequence encoding hUIFN α 80A2;

(2) a fragment containing a base sequence encoding glucagon; and

(3) a Trp pAT vector.

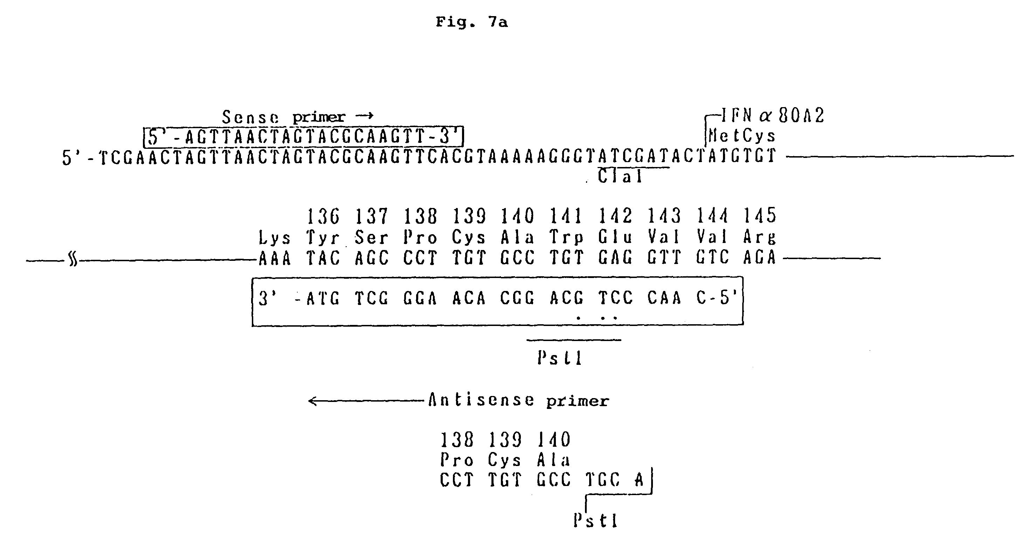

As shown in Figure 7a, the DNA fragment (1) is obtained by the polymerase chain reaction (PCR) method (Saiki et al., Science, 239, p. 487, 1988), with Trp pBR huIFN α 80A2 (Japanese Laid-Open Patent Publication No. 61-56199) as a template. For this purpose, first, two single-stranded oligonucleotides are chemically synthesized, i.e., the 20-nucleotide sense primer (VIII) and 25-nucleotide antisense primer (IX) shown in the following formulae.

Sense primer:

$$\text{5'-AGTTAACTAGTACGCAAGTT-3'} \qquad (VIII)$$

Antisense primer:

$$\text{5'-CAACCCTGCAGGCACAAGGGCTGTA-3'} \qquad (IX)$$

As shown in Figure 7a, the sense primer (VIII) corresponds to the rear part of the tryptophan promoter, while the antisense primer (IX) corresponds to the rear part of hUIFN α 80A2. Using these primers, a DNA fragment containing a sequence encoding hUIFN α 80A2 is obtained by amplification of the fragment with Taq polymerase. This DNA sequence is cleaved with ClaI and PstI, thus obtaining a ClaI-PstI fragment. This ClaI-PstI fragment contains a gene sequence encoding human IFN α 80A2 up through the alanine residue at the 140th position.

The DNA fragment (2) is the smaller of the fragments resulting from cleavage of the expression plasmid Trp pAT hUIFN γ/glucagon (Figure 2c), obtained in item (a) above, with PstI and BamHI, as shown in Figure 6. This PstI-BamHI fragment possesses a DNA base sequence encoding cysteine, serine and glutamic acid as well as a DNA sequence encoding glucagon.

The DNA fragment (3) is the larger of the fragments resulting from cleavage of the expression plasmid Trp pAT huIFN γ/glucagon (Figure 2c), obtained in item (a) above, with ClaI and BamHI, as shown in Figure 6. This ClaI-BamHI fragment contains the base sequence of the tryptophan promoter.

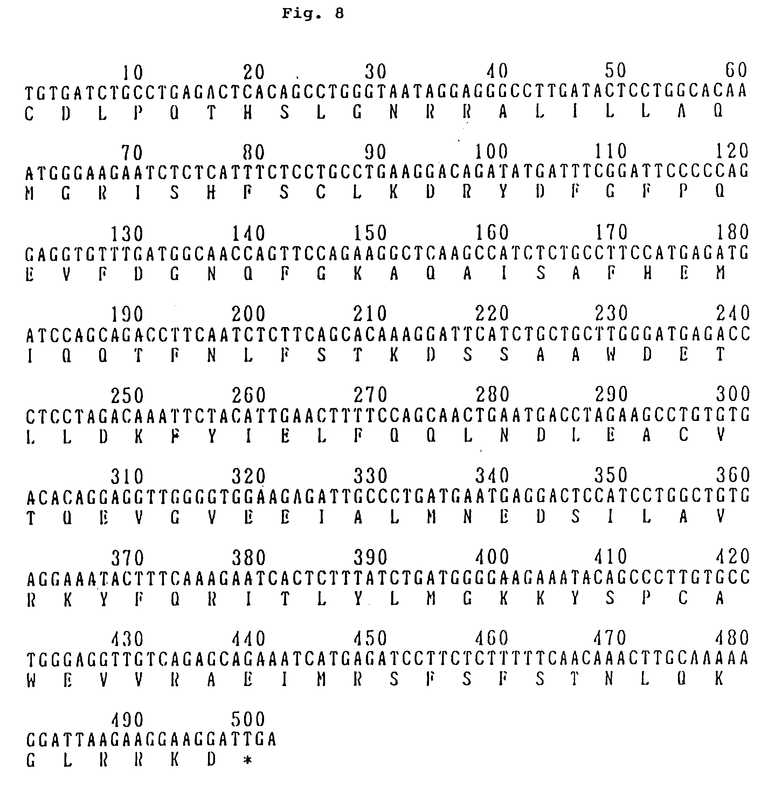

The expression vector Trp pAT huIFNα80A2/glucagon is obtained by ligating the aforementioned ClaI-PstI fragment (1), PstI-BamHI fragment (2) and ClaI-BamHI fragment (3) in the conventional manner using T4 DNA ligase (Figure 6). This vector Trp pAT huIFNα80A2/glucagon possesses a gene sequence encoding the segment of human IFNα80A2 up to the alanine (Ala) residue at the 140th position under the control of the tryptophan promoter, this sequence being adjoined on the downstream side by the sequence indicated in the lower right-hand portion of Figure 6. The entire sequence of huIFNα80A2 is shown in Figure 8.

(e) Construction of Trp pAT ΔCysII huIFNγ/glucagon

As shown in Figure 7c, the expression vector Trp pAT ΔCysII huIFNγ/glucagon of the present invention is formed from the expression vector Trp pAT ΔCys huIFNγ/glucagon described in the preceding item (b). That is, the said vector is obtained by converting the base sequence CTGCAG, which constitutes the only one PstI cleavage site in this plasmid, into CTCGAG by the PCR method (supra), using an oligonucleotide containing a mismatch. Specifically, the DNA fragment (1) encoding glucagon and adjoining sequences and the larger fragment (2) of the Trp pAT ΔCys huIFNγ vector (both to be described below) are ligated with T4 DNA ligase to obtain Trp pAT ΔCysII huIFNγ/glucagon.

First, two single-stranded oligonucleotides are chemically synthesized, i.e., the 21-nucleotide sense primer (X) and 21-nucleotide antisense primer (XI) shown by the following formulae:

Sense primer:

<div align="center">5'-CCGTTTCGCTCGAGCGAACAC-3' (X)</div>

Antisense primer:

<div align="center">5'-AGCTCTAGAGCTTTTATTAGG-3' (XI)</div>

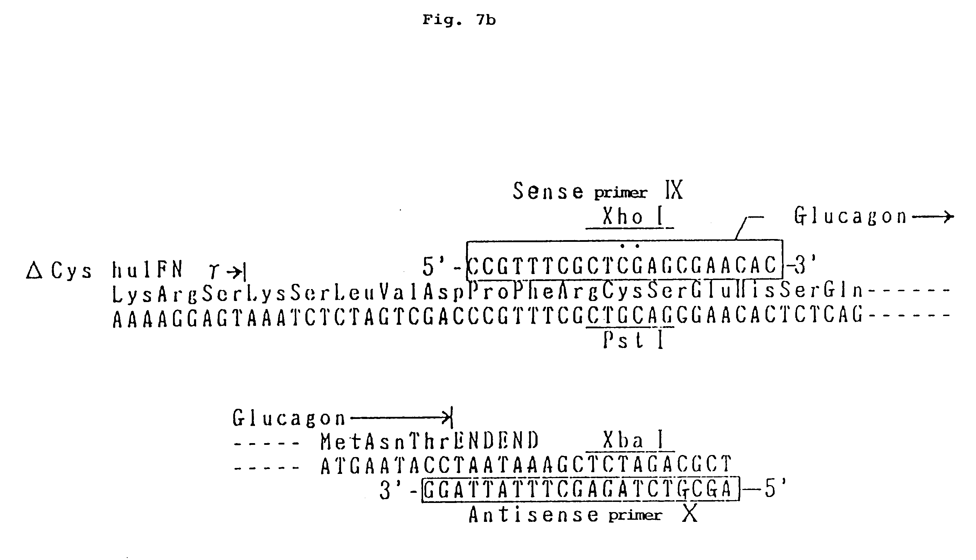

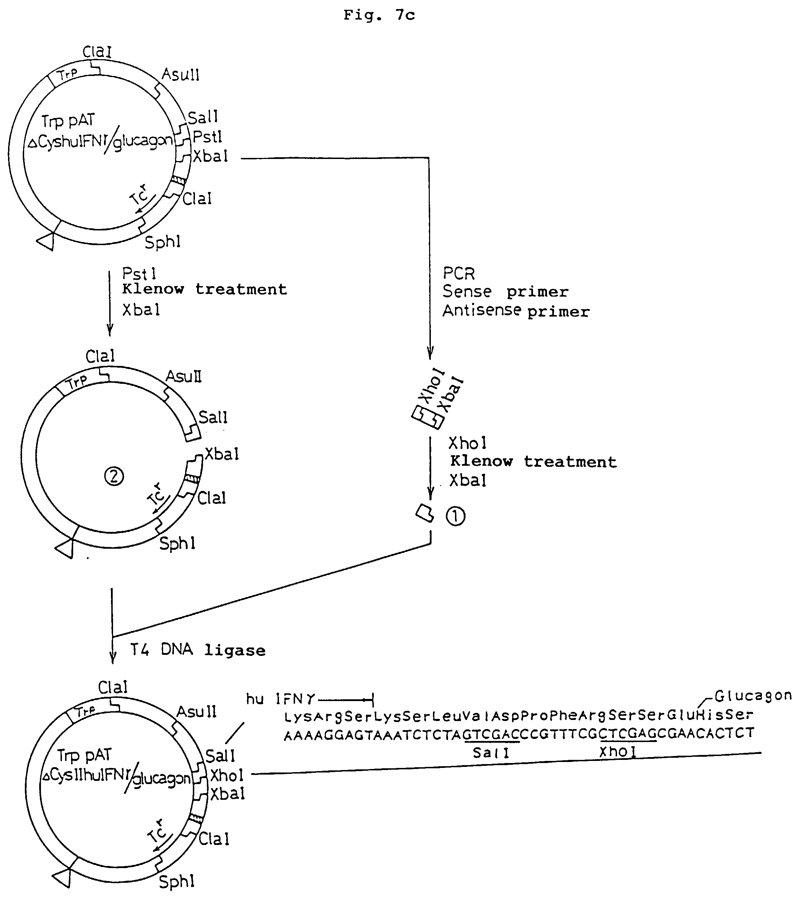

As shown in Figure 7b, the sense primer (X) corresponds to a sequence at the junction of huIFN $\gamma$ and glucagon, while the antisense primer (XI) corresponds to a sequence starting from the carboxyl terminus region of the glucagon and including a portion of the downstream region.

A DNA fragment (1) encoding glucagon and adjoining sequences is obtained as follows. Using the aforementioned two primers, a DNA fragment encoding glucagon and adjoining sequences is amplified by the above-mentioned PCR method with Trp pAT $\Delta$ Cys huIFN $\gamma$/glucagon as a template. Since the sense primer (X) contains a mismatch, the base sequence of the amplified DNA fragments differs in part from that of the template vector. Thus, the PstI cleavage site (CTGCAG) which is present in the base sequence encoding the region joining interferon-$\gamma$ and glucagon is converted into an XhoI cleavage site (CTCGAG) in the amplified DNA sequence. As a result, the cysteine codon TGC is converted into TCG, which encodes a serine residue. The amplified DNA fragments are cleaved with XhoI, and the cleaved ends are blunted with Klenow fragment. Next, this is cleaved with XbaI, thereby obtaining the DNA fragment (1).

As shown in Figure 7c, the larger fragment (2) of the Trp pAT $\Delta$Cys huIFN$\gamma$ vector is obtained by cleavage of the vector Trp pAT $\Delta$Cys huIFN$\gamma$/glucagon with PstI, followed by treatment with Klenow fragment. This sequence is then cleaved with XbaI, and the larger of the resulting fragments is the required fragment (2).

The expression vector Trp pAT $\Delta$CysII huIFN-$\gamma$/glucagon is then obtained by ligating the aforementioned Xho/Klenow-XbaI fragment (1) and PstI/Klenow-XbaI fragment (2) with T4 DNA ligase in the conventional manner.

This vector Trp pAT $\Delta$CysII huIFN$\gamma$/glucagon possesses a gene sequence encoding the segment of human interferon-$\gamma$ from the glutamine residue at the 4th position up through the serine residue at the 135th position under the control of the tryptophan promoter, this sequence being adjoined on the downstream side by the sequence shown in the lower right-hand portion of Figure 7c.

<u>Transformation of host cells and expression</u>

The aforementioned expression plasmids, i.e., (a) Trp pAT huIFN$\gamma$/glucagon, (b) Trp pAT $\Delta$Cys huIFN$\gamma$/glucagon, (c) Trp pAT $\Delta$Cys short huIFN$\gamma$/glucagon, (d) Trp pAT huIFN$\alpha$80A2/glucagon and (e) Trp pAT $\Delta$CysII huIFN$\gamma$/glucagon, are introduced into suitable host cells in accordance with the methods disclosed in "Molecular Cloning" (supra). The transformants are selected for tetracycline resistance. The transformants obtained by introduction of the aforementioned expression plasmids (a), (b), (c) and (e) produce a fusion protein containing human glucagon and human interferon-$\gamma$ or an analogues thereof. The transformants obtained by introduction of the aforementioned expression plasmid (d) produce a fusion protein containing human glucagon and IFN$\alpha$80A2. The applicable host organisms include E. coli K12 strains C600, JM103, AG-1, etc. The strain K12 C600 is especially suitable, and the use of this strain permits particularly efficient production of fusion proteins containing human glucagon.

A transformant obtained by introduction of the aforementioned expression vector Trp pAT huIFN$\gamma$/glucagon into the E. coli K12 strain C600 was designated Trp pAT huIFN$\gamma$ fused glucagon/C600, and deposited with the Fermentation Research Institute Agency for Industrial Science and Technology, 1-3, Higashi 1 chome, Tsukuba-shi, Ibaraki-ken, Japan on January 20, 1989 under Accession number FERM BP-2250, in accordance with the Budapest Treaty.

After cultivation of the transformants of the present invention and collection of the cell, the western blotting analysis employing SDS-polyacrylamide gel electrophoresis (SDS-PAGE) and anti-glucagon polyclonal antibodies confirmed that human glucagon is indeed expressed in the host cells in the form of a fusion protein. That is, SDS-PAGE revealed approximately 21-kilodalton and approximately 14-kilodalton bands corresponding to fusion proteins with human interferon-$\gamma$ and interferon-$\alpha$, respectively. The immunological reaction of these unmodified fusion proteins with antiglucagon polyclonal antibodies was confirmed by western blotting analysis. Using the method of the present invention, one liter of the culture mixture in which the transformants had been grown yielded approximately 3.06 g, 2.43 g, 3.64 g, 0.24 g and 2.45 g of fusion protein when the aforementioned expression vectors (a), (b), (c), (d) and (e), respectively, were used to transform the host.

<u>Solubilization of fusion protein, hydrolysis by V8 protease, isolation and purification of glucagon</u>

In order to recover glucagon from the aforementioned fusion proteins (X-Glu-Glucagon), since the fusion protein is accumulated in the host cells in the form of granular protein, this granular protein is first recovered and solubilized.

To accomplish this, for example, the cells are first collected by centrifuging the culture mixture in the conventional manner, then the cells are crushed and pelleted. These pellets are solubilized by addition of a denaturant or surfactant. The denaturants applicable for this purpose include guanidine (guanidine chloride), urea, etc. This denaturant is used after addition to a buffer solution in sufficient amount to solubilize the pellets. Saturated solutions of guanidine or urea can be used, but ordinarily concentrations in the range 6-8M are preferable. Tween 80 (trademark) is an appropriate surfactant for this present purpose, and should ordinarily be used at concentrations from 0.05 to 0.2% by weight. Taking into consideration the following process of enzymatic treatment, the pH of the buffer solution should preferably be set in the vicinity of the optimal pH of the enzyme to be used in the said process. For example, when protease V8 (Sigma Chemical Co., No. P-8400), derived from Staphylococcus aureus, is used as the enzyme, then a pH of 7.8 is desirable, and therefore a buffer solution with a strong buffering capacity in the vicinity of pH 7.8, for example, an ammonium hydrogencarbonate buffer, should be used in such cases. If the enzyme to be used in the enzymatic treatment contains impurities such as neutral enzymes, etc., then an enzyme inhibitor such as EDTA can also be added to the said buffer solution in order to inhibit the activity of the said impurities.

The mixture containing solubilized material obtained in this manner contains denaturants or surfactants in a concentration sufficiently high as to impede the action of the enzyme to be used in the following enzymatic treatment. In such cases, the mixture should ordinarily be diluted with a suitable diluent, such as the aforementioned buffer solution without denaturants or surfactants. The degree of dilution should be such that the solubilized state of the protein can be maintained and such that the enzymes to be used in the enzymatic treatment described below can act with sufficient effectiveness. For example, when guanidine or urea is used as the denaturant, then the mixture should be diluted so that the concentration of the said denaturant should be 4 M or lower, and preferably about 2 M or lower. However, it should be noted that the protein concentration in the mixture containing solubilized material should be 1-5 mg/ml, and more preferably 2-3 mg/ml.

Next, the glucagon is separated from the protein X by enzymatic treatment. The target glucagon is present in a partially denatured form fused with the protein X via an interposed glutamic acid residue. Since glucagon itself contains no glutamic acid residues, the glucagon can be released from the fusion protein in complete form by treatment with an enzyme which specifically cleaves the peptide bond at the carboxyl terminus of the glutamic acid residue. Enzymes suitable for this purpose include protease V8 (Sigma Chemical Co., No. P-8400), derived from Staphylococcus aureus, but this is by no means the only choice, and in principle any enzyme which specifically cleaves the peptide bond at the carboxyl terminus of the glutamic acid residue could be employed for the present purpose. For example, protease V8 derived from Staphylococcus aureus, ATCC No. 12600 can be preferably used. The amount of enzyme used is 1/50 to 1/1,000 (w/w), preferably about 1/500 (w/w), with respect to the total amount of protein in the aforementioned diluted cell preparation. The reaction mixture obtained by adding this enzyme to the aforementioned diluted cell preparation in this manner is allowed to react while gently stirring the mixture at a temperature between ordinary room temperature to 40°C for a period of 30 minutes to 7 hours. The reaction time varies according to the amount of enzyme added. For example, if the enzyme has been added in a proportion of approximately 1/500 (w/w), then a reaction time of approximately 4 hours is desirable.

The enzymatic reaction is terminated by addition of an acid such as hydrochloric acid, acetic acid, etc., preferably reducing the pH to about 3. The enzymatic reaction mixture so obtained contains the final product, glucagon, and intermediates of lower molecular weight than that of the X-Glu-Glucagon fusion protein. These intermediates presumably arise from cleavage of glutamic acid residues within the protein X. These intermediates also remain soluble even if the denaturants and surfactants contained in the mixture are removed. The following factors, e.g., may possibly contribute to the generation of these intermediates.

(i) A transient denaturation of the enzyme contained in the aforementioned reaction mixture by the denaturant or surfactant, resulting in pronounced reduction in enzymatic activity,

(ii) Inhibition of enzymatic action by the decomposition products of low molecular weight produced by the cleavage of the glutamic acid residues within the protein X, or

(iii) The target cleavage site at the target glutamic acid residue in the fusion protein becomes less susceptible to the enzymatic action because of folding or other changes in the higher order structure of the protein.

The yield of glucagon can be raised by further treatment and cleavage of these intermediates. An effective means of facilitating this includes the elimination from the enzymatic reaction mixture of the denaturant or surfactant as well as the low molecular weight decomposition products formed by cleavage of glutamic acid residues within the protein X. The elimination of these substances can be efficiently achieved by the desalinizing procedures such as gel filtration, dialysis, etc., gel filtration being a particularly suitable method. Gel filtration should preferably be performed under conditions such that, in the process of eliminating the denaturant or surfactant and the low molecular weight decomposition products, the enzyme and the intermediates are eluted in the same fraction. The fractions containing the intermediates and enzyme obtained by gel filtration are collected, and by adjusting the pH to the optimal pH of the enzyme, the enzyme is denatured and allowed to act upon the solubilized intermediates. The glucagon is then released by conducting an enzymatic reaction under the same conditions as mentioned above.

The glucagon cleaved in this manner from the fusion protein is purified by an appropriate combination of conventional techniques, e.g., various forms of chromatography such as gel filtration chromatography, affinity chromatography, ion exchange chromatography, hydrophobic gel chromatography, or centrifugal separation procedures, etc.

The method for recovering glucagon described above requires a comparatively small amount of enzyme to cleave the glutamic acid residues and is therefore economical.

Using the method of the present invention, one liter of the culture medium in which the transformants had been grown yielded approximately 150 mg, 160 mg and 170 mg of purified glucagon when the aforementioned expression vectors (a), (b) and (e), respectively, were used to transform the host. Purified glucagon was also obtained in the same manner from the fusion protein which was expressed when the transformant carrying the expression vector (c) or (d) was used. The degree of purity, amino acid composition, amino acid sequence and biological activity of the purified glucagon obtained in the above-mentioned manner were examined and the results confirmed that this substance was indeed human glucagon, containing no impurities and completely identical with natural glucagon as regards amino acid composition as well as biological activity. The glucagon obtained by the process described above can therefore be used in entirely the same manner as conventional types of glucagon preparations for purposes such as testing for growth hormone secretion, diagnosis of insulinoma, glycogen storage testing, emergency treatment of hypoglycemia, pretreatment in radiographic and endoscopic examination of the digestive tract and other clinical applications.

The present invention is not limited to the methods for the production of human glucagon in the form of a fusion protein. The present invention also includes vectors for the expression of the said fusion protein and transformants obtained by introduction of these vectors.

Thus, the present invention provides a method, as described above, for obtaining glucagon identical with natural glucagon from fusion proteins of the form X-Glu-Glucagon by a simple, efficient and economical process, using only small amount of enzymes. This glucagon is produced by utilization of recombinant DNA techniques, and can therefore be mass-produced at low cost. Moreover, since the amino acid sequence of the present type of product is completely identical with that of natural human glucagon, clinical administration entails no risk of allergic reactions, and the product can therefore be used in entirely the same manner as the glucagon preparations heretofore conventionally employed in clinical practice.

Example

Experiments of gene recombination described below were performed in accordance with the methods indicated in "Molecular Cloning" (Cold Spring Harbor Laboratory, 1982). All of the enzymes used were purchased from Takara Shuzo Co., Ltd.

(A) Design and chemical synthesis of DNA sequence encoding glucagon

The DNA sequence encoding the amino acid sequence of human glucagon was designed using, in principle, the codons utilized with greatest frequency by E. coli. The DNA sequence so designed, encoding the amino acid sequence from the initial histidine residue to the 29th threonine residue, is shown in Figure 1.

The fifteen short-chain fragments indicated in Figure 1 were chemically synthesized with an automatic nucleic acid synthesizer. These fragments were then annealed, thereby obtaining the DNA sequence with the double-stranded structure shown in Figure 1. This DNA sequence possesses an ATG codon encoding methionine as well as an AluI cleavage site at the 5′ terminus, and two TAA termination codons and a HindIII cleavage site at the 3′ terminus.

(B) Construction of expression vector

Five plasmid vectors capable of expressing human glucagon in E. coli, i.e., (a) Trp pAT huIFNγ/glucagon, (b) Trp pAT ΔCys huIFNγ/glucagon, (c) Trp pAT ΔCys short huIFNγ/glucagon, (d) Trp pAT huIFNα80A2/glucagon and (e) Trp pAT ΔCysII huIFNγ/glucagon, were constructed by the following procedure.

(a) Construction of Trp pAT huIFNγ/glucagon

This expression vector was constructed by ligating the three DNA fragments obtained in items (1)-(3) below. The construction of this expression vector is schematically shown in Figures 2a-2c.

(1) A 19 bp PstI-KpnI fragment containing the base sequence encoding the front half of the amino acid sequence of glucagon was prepared as follows. This base sequence was designed by adding the codon GAA for glutamic acid and a PstI cleavage site to the 5′ terminus of the DNA sequence encoding the front half of the glucagon consisting of histidine, serine, glutamine, glycine and a portion of a threonine codon, and providing a KpnI cleavage site at the 3′ terminus. This base sequence is shown in formula (IV) of the preceding text. Then, the two 19-mer oligonucleotides to form this base sequence were chemically synthesized and annealed, thus obtaining a 19 bp fragment containing a base sequence encoding the front half of the amino acid sequence of glucagon as well as an PstI cleavage site on the upstream side and a KpnI cleavage site on the downstream side.

(2) A KpnI-HindIII/Klenow fragment of the plasmid pGA1 was prepared as follows. As shown in Figure 2a, using the plasmid ptac12 (supra) as prototype, the cloning vector pGA1 was constructed as follows. First, the 15 bp synthetic linker shown by the foregoing formula (V) was inserted into the PvuII cleavage site of ptac12, thereby constructing a plasmid (ptac12/SL1) with a HindIII cleavage site downstream to the PvuII cleavage site. This plasmid ptac12/SL1 was then cleaved with PvuII and HindIII, and the synthetic gene of approximately 100 bp obtained in the preceding item (A) (i.e., the DNA sequence shown in Figure 1) was inserted, thus obtaining a plasmid designated as pGA1.

Next, as shown in Figure 2c, the plasmid pGA1 was digested with HindIII and treated with Klenow fragment to blunt the cleaved ends, after which the cut plasmid was digested with KpnI, and the shorter of the fragments so obtained, i.e., a 90 bp KpnI-HindIII/Klenow fragment, was recovered. As shown in Figure 1, this KpnI-HindIII/Klenow fragment encodes the rear half of the amino acid sequence of glucagon, starting from the KpnI cleavage site at an intermediate position in the aforementioned base sequence encoding the amino acid sequence of glucagon.

(3) An XbaI-PstI fragment of the plasmid vector Trp pAT huIFNγ/Kallikrein was prepared in the following manner. As shown in Figure 2b, an expression plasmid for a fusion protein formed by human interferon-γ and PSTI (Trp pAT huIFNγ/PSTI, J. Biochem., 102, 607-612 1987 (supra)) was digested with SalI at 37°C for 1 hour, after which the product was treated with BAP at 37°C for 2 hours, and the larger fragment (SalI-SalI fragment) was isolated. In a separate operation, the plasmid Trp pAT huIFNγ was digested with SalI and XbaI and the smaller XbaI-SalI fragment was recovered. Then, the aforementioned SalI-SalI fragment, the aforementioned XbaI-Sal fragment and the synthetic oligonucleotide adapter indicated by formula (VI) in the foregoing text (containing a DNA sequence encoding the recognition site (Phe-Arg) of human plasma kallikrein) were ligated with T4 DNA ligase.

The plasmid vector obtained in this manner was designated as Trp pAT huIFNγ/Kallikrein. This vector Trp pAT huIFNγ/Kallikrein contains a gene sequence encoding the segment of human interferon-γ up through the serine residue in the 135th position under the control of the tryptophan promoter, followed on the downstream side by the sequence shown in the lower part of Figure 2b. After cleavage of this vector Trp pAT huIFNγ/Kallikrein with XbaI as shown Figure 2c, the cleaved ends were blunted with Klenow fragment. Next, this fragment was cleaved with PstI, and the larger of the resulting DNA fragments (XbaI-PstI fragment, 3.6 kbp) was isolated.

The aforementioned DNA fragments (1)-(3) (each having been isolated by agarose gel electrophoresis in accordance with the method indicated in Nucleic Acids Res., 8, 253-264, 1980) were ligated with T4 DNA ligase as shown in Figure 2c, thus obtaining an expression vector which was designated as Trp pAT huIFNγ/glucagon. Next, the base sequence of the DNA fragment which had been introduced into this expression plasmid was examined in accordance with the method indicated in Proc. Natl. Acad. Sci. U. S. A., 74, 5464-5467, 1977, thereby verifying that the said DNA fragment does indeed correctly encode the entire 29 amino acid sequence of human glucagon.

(b) Construction of Trp pAT ΔCys huIFNγ/glucagon

This expression vector was constructed by ligating the three DNA fragments obtained as indicated in items (1)-(3) below. The construction of this expression vector is schematically shown in Figures 4a and 4b.

(1) A ClaI-AsuII fragment of the human interferon-γ expression plasmid Trp pAT ΔCys huIFNγ was prepared in the following manner. The said human interferon-γ expression plasmid Trp pAT ΔCys huIFNγ was constructed with Trp pAT huIFNγ (supra) as prototype, as shown in Figure 4a. First, Trp pAT huIFNγ was digested with ClaI, AvaII and SphI, thus obtaining a ClaI-SphI fragment and an AvaII-SphI fragment. In addition, the aforementioned synthetic oligonucleotide adapter (VII) was synthesized. Then, this ClaI-SphI fragment, AvaII-SphI fragment and synthetic adapter (VII) were ligated with T4 DNA ligase. The plasmid obtained in this manner was designated as Trp pAT ΔCys huIFNγ.

Next, as shown on the right-hand side of Figure 4b, this plasmid Trp pAT ΔCys huIFNγ was digested with ClaI and AsuII at 37°C for 1 hour, and a 275 bp fragment (ClaI-AsuII fragment) was isolated from the product.

(2) A AsuII-SphI fragment of the expression plasmid Trp pAT huIFNγ/glucagon obtained in item (a) above was prepared by digesting Trp pAT huIFNγ/glucagon with AsuII and SphI at 37°C for 1 hour and then isolating the smaller fragment.

(3) Furthermore, a ClaI-SphI fragment of the expression plasmid Trp pAT huIFNγ/glucagon obtained as in item (a) above was prepared by digesting Trp pAT huIFNγ/glucagon with ClaI and SphI at 37°C for 1 hour and then isolating the larger fragment.

The aforementioned DNA fragments (1)-(3) (each having been isolated by agarose gel electrophoresis in accordance with the method indicated in Nucleic Acids Res., 8, 253-264, 1980) were ligated with T4 DNA ligase as shown in Figure 4b, thus obtaining an expression vector which was designated as Trp pAT ΔCys huIFNγ/glucagon. Next, the base sequence of the DNA fragment which had been introduced into this expression plasmid Trp pAT ΔCys huIFNγ/glucagon was examined in accordance with the method indicated in Proc. Natl. Acad. Sci. U. S. A., 74, 5464-5467, 1977), thereby verifying that the said DNA fragment did indeed correctly encode the entire 29 amino acid sequence of human glucagon.

(c) Construction of Trp pAT ΔCys short huIFNγ/glucagon

An expression vector was constructed in the following manner. The procedure for construction of this expression vector is schematically shown in Figure 5.

The expression plasmid Trp pAT ΔCys huIFNγ/glucagon obtained in item (b) above was digested with AsuII and PstI at 37°C for 1 hour, and the cleaved ends were blunted by treatment with Klenow fragment. The larger of the resulting fragments (AsuII/Klenow-PstI/Klenow fragment) was then religated with T4 DNA ligase, thus obtaining an expression vector which was designated as Trp pAT ΔCys short huIFNγ/glucagon. Next, the base sequence of the DNA fragment which had been introduced into this expression plasmid Trp pAT ΔCys short huIFNγ/glucagon was examined in accordance with the method indicated in Proc. Natl. Acad. Sci. U. S. A., 74, 5464-5467, 1977), thereby verifying that the said DNA fragment did indeed correctly encode the entire 29 amino acid sequence of human glucagon.

(d) Construction of Trp pAT huIFNγ80A2/glucagon

This expression vector was constructed by ligating the three DNA fragments obtained as indicated in items (1)-(3) below. The construction of this expression vector is schematically shown in Figure 6.

(1) A ClaI-PstI fragment containing a base sequence encoding huIFNα80A2 was prepared as follows. First, two single-stranded oligonucleotides were synthesized, i.e., a 20-nucleotide sense primer (formula (VIII) above) corresponding to the latter portion of the Trp promoter of the human IFNα80A2 expression plasmid Trp pBR huIFN 80A2 (supra) and a 25-nucleotide antisense primer (formula (IX) above) encoding the latter portion of huIFNα80A2. Using these primers and Taq polymerase, a DNA fragment containing a base sequence encoding huIFN 80A2 was obtained by the amplification of the fragment in accordance with the PCR method (supra). This amplified fragment was digested with ClaI and PstI at 37°C for 1 hour, and the resulting ClaI-PstI fragment was isolated.

(2) A PstI-BamHI fragment containing a base sequence encoding glucagon was prepared by digesting the expression plasmid Trp pAT huIFNγ/glucagon obtained in item (a) above with PstI and BamHI at 37°C for 1 hour and isolating the smaller fragment (Figure 6).

(3) Likewise, a ClaI-BamHI fragment of the plasmid vector Trp pAT huIFNγ/glucagon was prepared by digesting the expression plasmid Trp pAT huIFNγ/glucagon obtained in item (a) above with ClaI and BamHI at 37°C for 1 hour and isolating the larger fragment (Figure 6).

The aforementioned DNA fragments (1)-(3) (each having been isolated by agarose gel electrophoresis in accordance with the method indicated in Nucleic Acids Res., 8, 253-264, 1980) were ligated with T4 DNA ligase as shown in Figure 6, thus obtaining an expression vector which was designated as Trp pAT huIFNα80A2/glucagon. Next, the base sequence of the DNA fragment which had been introduced into this expression plasmid Trp pAT huIFNα80A2/glucagon was examined in accordance with the method indicated in Proc. Natl. Acad. Sci. U. S. A., 74, 5464-5467, 1977), thereby verifying that the said DNA fragment did indeed correctly encode the entire 29 amino acid sequence of human glucagon.

(e) Construction of Trp pAT ΔCysII huIFNγ/glucagon

This expression vector was derived from the plasmid Trp pAT ΔCys huIFNγ/glucagon obtained in item (b) above. The construction of the said expression vector is schematically shown in Figure 7c.

First, a 21-nucleotide sense primer (formula (X) above) and a 21-nucleotide antisense primer (formula (XI) above) were synthesized. Using these primers and Taq polymerase with Trp pAT ΔCys huIFNγ/glucagon as template, an approximately 130 base pair DNA fragment was amplified in accordance with the PCR method. This amplified DNA fragment was digested with XhoI at 37°C for 1 hour, treated with Klenow fragment, and then digested with XbaI at 37°C for 1 hour, thereby obtaining a DNA fragment (1) with approximately 110 base pairs.

Next, a DNA fragment (2) was prepared from the vector Trp pAT ΔCys huIFNγ/glucagon by digesting the said vector Trp pAT ΔCys huIFNγ/glucagon with PstI 37°C for 1 hour, followed by treatment with Klenow fragment, then digesting with XbaI at 37°C for 1 hour and recovering the large fragment (2).

The expression vector Trp pAT ΔCysII huIFNγ/glucagon was constructed by ligating the DNA fragments obtained above with T4 DNA ligase. Next, the base sequence of the DNA fragment which had been introduced into this expression vector was examined in accordance with the method indicated in Proc. Natl. Acad. Sci. U. S. A., 74, 5464-5467, 1977, thereby verifying that the said DNA fragment did indeed correctly encode the entire 29 amino acid sequence of human glucagon.

(C) Transformation of host cells and gene expression

Using E. coli K12 strain C600 (F⁻, thi⁻1, thr⁻1, leuB6, lacY1, tonA21, supE44) as host cells, transformation was effected with each of the expression plasmids constructed in the manner described above, i.e., (a) Trp pAT huIFNγ/glucagon, (b) Trp pAT ΔCys huIFNγ/glucagon, (c) Trp pAT ΔCys short huIFNγ/glucagon, (d) Trp pAT huIFNα80A2/glucagon and (e) Trp pAT ΔCysII huIFNγ/glucagon. Since the transformants into which these expression plasmids were introduced all acquired tetracycline resistance, the cells were cultivated overnight at 37°C on a medium containing tetracycline (LB plate medium containing 15 μg/ml of tetracycline), and the cells which formed colonies were selected. Next, the colonies were further cultivated in a modified M9 medium for at 37°C for 24 hours, after which the cells were collected by centrifugation and suspended in a buffer solution (0.1 M Tris-HCl, 1% SDS, 20% sucrose and 1% β-mercaptoethanol, pH 6.8) suitable for SDS gel analysis. This suspension was subjected to SDS-PAGE, and the production of a fusion protein containing glucagon was examined by staining the gel with Coomassie brilliant blue R as well as by western blotting analysis using anti-glucagon polyclonal antibodies. The results revealed that a granular protein of molecular weight approximately 21 kilodaltons was produced in the hosts transformed with the aforementioned expression plasmids (a), (b), (d) and (e), while the hosts transformed with the aforementioned expression plasmid (c) produced a granular protein of approximately 14 kilodaltons, moreover, the said proteins in that form without further modification reacted with anti-glucagon antibodies. Thus, the results confirmed that all the transformants produced a fusion protein containing the desired glucagon.

(D) Solubilization of fusion protein, hydrolysis by V8 protease; isolation and purification of glucagon

After the host cells transformed with the aforementioned expression vectors (a), (b), (c), (d) and (e) had been cultivated in modified M9 medium at 37°C for 24 hours, the cells were collected by centrifugation and suspended in buffer solution A (8 g/ℓ sodium chloride, 0.2 g/ℓ potassium chloride, 2.9 g/ℓ disodium hydrogenphosphate·12H₂O, 0.2 g/ℓ potassium dihydrogenphosphate, 5 mM EDTA, 10 mM β-mercaptoethanol and 10 mM benzamidine, pH 6.65). After stirring thorough, the suspension was centrifuged and the cells so obtained were washed. The cells were then resuspended in the aforementioned buffer solution A. Then, lysozyme was added so as to achieve a final concentration of 2 mg per gram of cells (wet weight), and the mixture was allowed to react at 35°C for 1 hour. Next, the reaction mixture was ultrasonicated and centrifuged, and the granular fraction was recovered in the form of a pelleted precipitate. The glucagon was finally isolated from these granular fraction pellets by either a one-stage or two-stage enzymatic reaction, to be described below in items (D-1) and (D-2), respectively.

(D-1) Isolation by one-stage enzymatic reaction

The granular fraction pellets obtained from host cells transformed by the aforementioned expression vector (a) were suspended in buffer solution A (containing 6 M guanidine hydrochloride), the amount of the said buffer solution being 1/6 of the initial volume of the cell culture used. After thorough stirring and ultrasonication, the mixture was left standing at room temperature for 2 hours to effect solubilization. Next, the fraction which had not been solubilized by this procedure was removed by centrifugation, and the supernate was then dialyzed overnight against buffer solution B (50 mM ammonium bicarbonate, 5 mM EDTA, pH 7.8) containing 2 M guanidine hydrochloride. The dialysate was then mixed with an equal amount of buffer solution B so as to achieve a guanidine hydrochloride concentration of 1 M, and the resulting specimen was designated as (a). SDS-PAGE analysis of specimen (a) revealed that 1 g (approximately 50 μmol) of human interferon-γ was produced per liter of culture broth. Protease V8 (Sigma Chemical Co., No. P-8400), derived from Staphylococcus aureus and specifically cleaving the peptide bonds of glutamic acid residues, was added to specimen (a) in a proportion of 1/10 (w/w), the proportion being based on the total amount of protein present in this mixture, and the mixture was allowed to react at 37°C for 1 hour. Since the desired glucagon is fused with interferon-γ via a glutamic acid residue, and since no glutamic acid residues are present within the glucagon molecule, this protease V8 treatment permits the separation of the entire glucagon molecule in complete and unmodified form. However, the specimen (b) obtained in this manner also contained an admixture of peptides of various lengths arising from decomposition of glucagon and human interferon-γ. In order to eliminate these various sizes of peptide impurities, specimen (b) was centrifuged, the precipitate was removed, and the supernate was subjected to reverse phase HPLC using a C₁₈ column (4.6 mm i.d. x 250 mm). The initial eluent used was a mixed 32% acetonitrile-0.1% trifluoroacetic acid solvent, and elution was performed while increasing the concentration of acetonitrile by 0.5% per minute from the instant 5 minutes after the start of elution until a concentration of 42% was achieved.

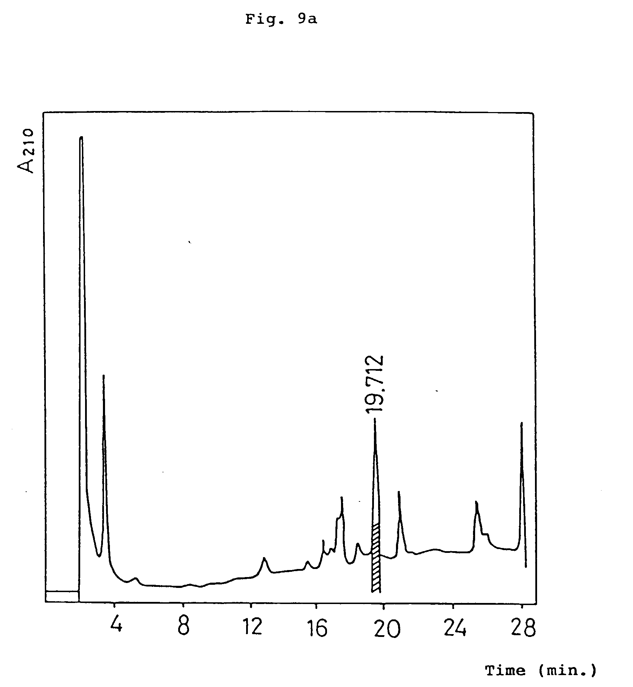

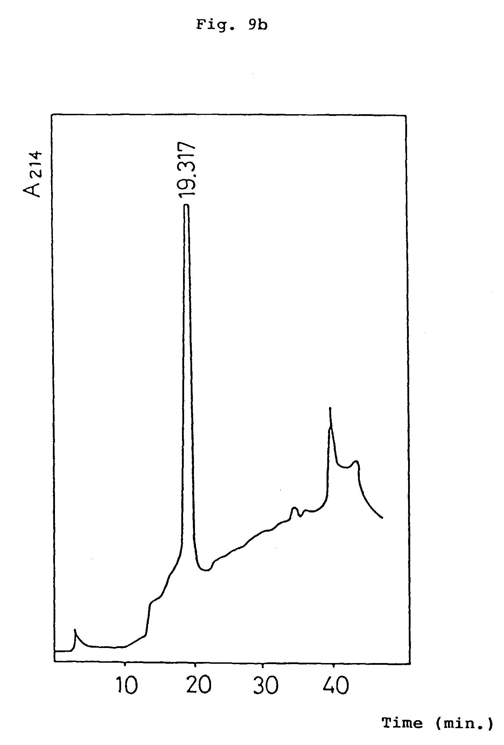

The results obtained are shown in Figure 9a. A glucagon standard was analyzed by HPLC under identical conditions, and was detected at a position corresponding to a retention time of 19.7 minutes. Therefore, the fraction eluted at the position corresponding to about 20 minutes in Figure 9a was collected, after which this fraction was purified by ion exchange HPLC. The fraction containing glucagon was introduced into a column (7.5 mm i.d. x 75 mm) packed with the anion exchange resin DEAE-5PW buffered with 10 mM Tris-HCl (pH 8.5) containing 20% acetonitrile (buffer solution C). Then, after washing with buffer solution C for 5 minutes, the material was eluted in buffer solution C under a linear concentration gradient of 0-0.1 M sodium chloride (i.e., sodium chloride concentration increased from zero to 0.1 M over a 30 minute period, with a flow rate of 1.0 ml/min). The result was as shown in Figure 9b. On the basis of the eluted position of the glucagon standard, the principal component of the analyte (retention time 19.3 minutes, specimen (b)) was confirmed as being glucagon. Specimen (b) was then subjected to amino acid composition analysis and amino acid sequencing by the methods described below. The results of these analyses are shown in Tables 1 and 2, respectively.

Analysis of amino acid composition

The analyte was desalinized by HPLC (Aquapore RP-300 column, Brownlee), and hydrolyzed with 4 M methanesulphonic acid (containing 0.2% 3-(2-aminoethyl) indole) at 110°C for 24 hours. The amino acid composition of this hydrolyzate was then determined by means of a Hitachi Model 835 amino acid analyzer.

Amino acid sequencing

After desalination by HPLC (Aquapore RP-300 column, Brownlee), the amino acid sequence of the analyte was determined using an Applied Biosystems 477A Protein Sequencer.

In the above-described purification process, approximately 33 mg (about 10 µmol) of purified glucagon was obtained from 1 g (approximately 50 µmol) of fusion protein, i.e., approximately 33 mg of purified glucagon was obtained per liter of culture broth. However, the present method of purification requires a large amount of enzyme to cleave the peptide bond of the glutamic acid residue.

Table 1

| Amino acid composition | | |
|---|---|---|
| Amino acid | Number of amino acid | |
| | Found | Calculated |
| Asp | 4.0 | 4 |
| Thr | 2.7 | 3 |
| Ser | 3.6 | 4 |
| Glu | 3.0 | 3 |
| Gly | 1.2 | 1 |
| Ala | 1.1 | 1 |
| Val | 1.0 | 1 |
| Met | 1.0 | 1 |
| Leu | 2.2 | 2 |
| Tyr | 2.0 | 2 |
| Phe | 2.0 | 2 |
| Lys | 1.2 | 1 |
| His | 1.0 | 1 |
| Arg | 1.9 | 2 |
| Trp | 1.1 | 1 |
| Total | | 29 |

Table 2

| Analysis of amino acid sequence | | |
|---|---|---|
| Degradation step | Amino acid residue | Recovery (%) |
| 1 | His | 9.6 |

Table 2 (continued)

| Analysis of amino acid sequence | | |
|---|---|---|
| Degradation step | Amino acid residue | Recovery (%) |
| 2 | Ser | 77.9 |
| 3 | Gln | 27.5 |
| 4 | Gly | 29.6 |
| 5 | Thr | 52.2 |
| 6 | Phe | 28.2 |
| 7 | Thr | 47.4 |
| 8 | Ser | 58.6 |
| 9 | Asp | 19.6 |
| 10 | Tyr | 16.1 |
| 11 | Ser | 42.5 |
| 12 | Lys | 8.7 |
| 13 | Tyr | 14.0 |
| 14 | Leu | 12.0 |
| 15 | Asp | 11.3 |
| 16 | Ser | 25.4 |
| 17 | Arg | 9.9 |
| 18 | Arg | 7.8 |
| 19 | Ala | 7.7 |
| 20 | Gln | 7.9 |
| 21 | Asp | 6.3 |
| 22 | Phe | 3.2 |
| 23 | Val | 2.9 |
| 24 | Gln | 6.6 |
| 25 | Trp | 1.0 |
| 26 | Leu | 1.8 |
| 27 | Met | 1.0 |
| 28 | Asn | 2.9 |
| 29 | Thr | 0.8 |

(D-2) Isolation by two-stage enzymatic reaction

Glucagon was prepared by the following process from the granular fraction pellets obtained from the E. coli transformed with each of the aforementioned expression vectors (a), (b), (c), (d) and (e). First, 1 g of the pelleted granular fraction was dissolved in 35 ml of buffer solution 1 (8 M guanidine hydrochloride, 50 mM ammonium bicarbonate, 5 mM EDTA and 10 mM DTT, pH 7.8). Next, 105 ml of buffer solution 2, not containing guanidine hydrochloride (50 mM ammonium bicarbonate, 5 mM EDTA and 10 mM DTT, pH 7.8) was added, thereby adjusting the concentration of guanidine hydrochloride of the solution to 2 M and the protein concentration to 2-3 mg/ml (measured with a Proteinassay Kit, BioRad Co.). Protease V8 (Sigma Chemical Co., No. P-8400), derived from Staphylococcus aureus, was then added to this solution in an amount equal to 1/500 (w/w) of the protein present in this mixture, and a reaction was conducted at 25°C while gently stirring the mixture. After 4 hours, the reaction was terminated by addition of hydrochloric acid, adjusting the pH of the solution to 3.0. The reaction products were examined by sampling the mixture both during and after termination of the enzymatic reaction and subjecting the samples to reverse phase HPLC using a Vydac Protein $C_4$ column (0.46 mm i.d. x 15.0 cm). The initial eluent used was a mixed 25% acetonitrile-0.1% trifluoroacetic acid solvent, and elution was performed by linearly increasing the concentration of acetonitrile up to 35% over a period of 25 minutes commencing from the start of elution. The results of these assays showed that intermediates arising from partial decomposition of the fusion protein as well as small quantities of glucagon were progressively generated as the reaction proceeded.

Accordingly, after the enzymatic reaction, the insoluble matter in the reaction mixture was removed by centrifugation, and the supernate was desalinized by gel filtration chromatography using a Sephadex G-15 column (5 cm i.d. x 30 cm, capacity 590 ml). The eluent used was 50mM acetic acid buffer solution (pH 3.3) containing 5 mM EDTA, and the fraction of high molecular weight containing the intermediates and protease V8 was recovered. This fraction was adjusted to pH 7.8 with ammonium hydroxide, and again a reaction was conducted at 25°C while gently stirring the mixture. Thus, the protease V8 in the reaction mixture was renatured and acted upon the intermediates, and within approximately 5 hours the glucagon was completely released from the said intermediates. Urea was then added to this reaction mixture so as to achieve a final urea concentration of 6 M, and the pH of the mixture was then adjusted to 5.0 with acetic acid, bringing the final volume to approximately 200 ml. This mixture was then subjected to ion exchange chromatography using a Sephadex column (2.0 cm i.d. x 20.0 cm, capacity 63 ml, fast flow type) equilibrated beforehand with buffer solution 3 (6 M urea solution adjusted to pH 5.0 with acetic acid). The nonadsorbed fraction was removed by washing the column successively with 120 ml of buffer solution 3 and then 120 ml of buffer solution containing 0.05 M sodium chloride. Next, elution was performed under a linear sodium chloride concentration gradient, increasing the sodium chloride concentration in buffer solution 3 from 0.05 to 0.15 M (using a volume of eluent equal to 15 times the capacity of the column), and the glucagon was eluted with the buffer containing approximately 0.1 M sodium chloride. The glucagon fraction (approximately 70 ml) was promptly desalinized by gel filtration chromatography using a Sephadex G-15 column equilibrated with 50 mM acetic acid (5 cm i.d. x 30 cm, capacity 590 ml). At this stage the purity of the glucagon was approximately 97-98%, and hence the product was further purified by fractionation with HPLC.

The fraction containing glucagon, obtained by gel filtration as described above, was introduced into a Vydac Protein $C_4$ column (2.2 cm i.d. x 25 cm, 10-15 μm packing pore size 300 Å) equilibrated with 25.0% acetonitrile-0.1% trifluoroacetic acid, where separation and purification of glucagon was effected by means of a linear concentration gradient, raising the acetonitrile concentration from 25 to 30% over a period of 120 minutes at a flow rate of 4 ml/min. The glucagon was thereby eluted about 90 minutes after commencement of the process. The yield of purified glucagon per gram of the pelleted granular fraction obtained in this manner was approximately 16.3 mg, approximately 21.9 mg and approximately 23.1 mg for E. coli transformed with the expression vectors (a), (b) and (e), respectively, while the corresponding yields per gram of fusion protein were 49.0 mg, 65.8 mg and 69.4 mg, respectively. Purified glucagon was also obtained in a similar manner when the expression vectors (c) and (d) were used for transformation.

Purified glucagon obtained from E. coli transformed with expression vector (a) was analyzed with respect to (i) purity, (ii) amino acid composition, (iii) amino acid sequence and (iv) biological activity using the following procedures.

(i) Purity

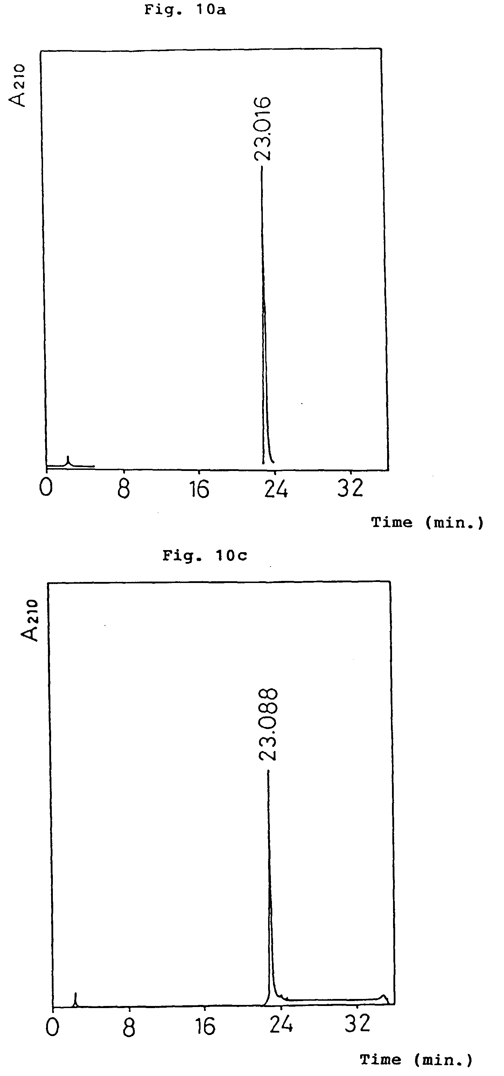

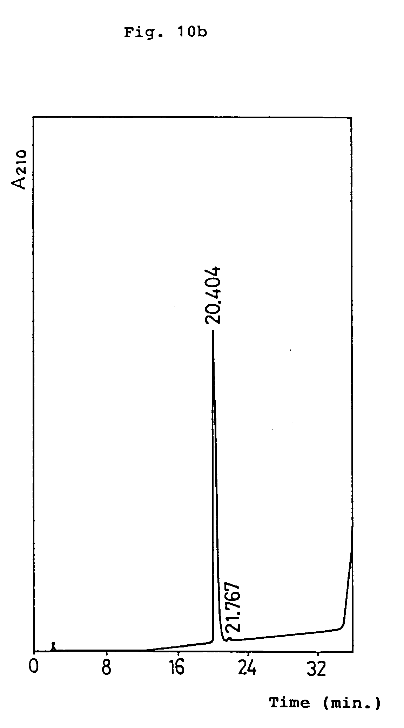

Purity was examined by reverse phase chromatography using a Vydac Protein $C_4$ column (0.46 cm i.d. x 15.0 cm). The initial eluent used was a mixed 25% acetonitrile-0.1% trifluoroacetic acid solvent, and elution was performed by linearly increasing the concentration of acetonitrile up to 35% over a period of 25 minutes commencing from the start of elution. The HPLC chart so obtained is shown in Figure 10a, while Figure 10c shows the corresponding chart for commercially available natural glucagon (Sigma Chemical Co.). As seen from Figure 10a, the elution chart of the present variety of purified glucagon displayed only a single peak, and therefore, this glucagon contained no impurities (i.e., a purity of 100%). Moreover, the retention time (23.016 minutes) for elution of the present variety of purified glucagon was almost identical with the corresponding time for natural glucagon as shown in Figure 10c, i.e., 23.088 minutes, demonstrating that the present variety of glucagon is indeed identical with the natural type. The HPLC chart of another sample of glucagon obtained according to the method mentioned above is shown in Figure 10b. The retention time for elution of the present variety of purified glucagon was 20.404 minutes. The purity of this glucagon was 99.3%. Analysis of the purified glucagon preparations obtained from the transformants created with the expression vectors (b)-(e) yielded essentially the same results.

(ii) Amino acid composition

Prior to analysis of amino acid composition, the analyte was hydrolyzed with 4 M methanesulphonic acid (containing 0.2% 3-(2-aminoethyl) indole) at 110°C for 24 hours. The amino acid composition of this specimen was then determined by means of a Hitachi Model 835 amino acid analyzer. The results of this analysis, shown in Table 3, demonstrate that the respective amino acid proportions of the purified glucagon obtained from E. coli transformed with the expression vector (a) were identical with the theoretical values. Analysis of the purified glucagon preparations obtained from the transformants created with the expression vectors (b)-(e) yielded essentially the same results.

(iii) Amino acid sequencing

The amino acid sequence of the purified glucagon obtained from the host cells transformed with expression vector (a) was determined using an Applied Biosystems 477A Protein Sequencer. The results of this analysis, shown in Table 4, demonstrate that the amino acid sequences of the purified glucagon obtained from E. coli transformed with the expression vector (a) were identical with that of natural glucagon. Analysis of the purified glucagon preparations obtained from the transformants created with the expression vectors (b)-(e) yielded essentially the same results.

Table 3

| Amino acid composition | | |
|---|---|---|
| Amino acid | Number of amino acid | |
| | Found | Calculated |
| Asp | 4.0 | 4 |
| Thr | 2.8 | 3 |
| Ser | 3.7 | 4 |
| Glu | 3.1 | 3 |
| Pro | 0.0 | 0 |
| Gly | 1.0 | 1 |
| Ala | 1.0 | 1 |
| Cys/2 | 0.0 | 0 |
| Val | 0.9 | 1 |
| Met | 1.0 | 1 |
| Ile | 0.0 | 0 |
| Leu | 2.1 | 2 |
| Tyr | 2.0 | 2 |
| Phe | 2.0 | 2 |
| Lys | 1.1 | 1 |
| His | 1.0 | 1 |
| Trp | 1.1 | 1 |
| Arg | 2.0 | 2 |
| Total | | 29 |

Table 4

| Analysis of amino acid sequence | | |
|---|---|---|
| Degradation step | Amino acid residue | Amount of amino acid detected (pmol) |
| 1 | His | 1333 |
| 2 | Ser | 885 |
| 3 | Gln | 1412 |
| 4 | Gly | 1597 |
| 5 | Thr | 1146 |
| 6 | Phe | 2473 |
| 7 | Thr | 550 |
| 8 | Ser | 333 |
| 9 | Asp | 975 |
| 10 | Tyr | 1162 |
| 11 | Ser | 357 |
| 12 | Lys | 658 |
| 13 | Tyr | 804 |
| 14 | Leu | 1023 |
| 15 | Asp | 701 |
| 16 | Ser | 213 |
| 17 | Arg | 529 |
| 18 | Arg | 551 |
| 19 | Ala | 779 |

Table 4   (continued)

| Analysis of amino acid sequence | | |
|---|---|---|
| Degradation step | Amino acid residue | Amount of amino acid detected (pmol) |
| 20 | Gln | 639 |
| 21 | Asp | 523 |
| 22 | Phe | 464 |
| 23 | Val | 537 |
| 24 | Gln | 178 |
| 25 | Trp | 109 |
| 26 | Leu | 356 |
| 27 | Met | 277 |
| 28 | Asn | 278 |
| 29 | Thr | 31 |
| Load = 3856 pmol | | |

(iv) Biological activity

As indices of biological activity, receptor assay using a rat liver membrane fraction, cAMP response assay using free rat liver cells and release of glucose by perfused liver specimens were investigated. The results showed that the purified glucagon obtained from E. coli transformed with the expression vectors (a)-(e) manifested the same activity as that of natural glucagon in all of these assays, thus confirming again that these products are identical with natural glucagon.

**Claims**

**Claims for the following Contracting States : DE, GB, FR, IT, NL, SE, LI, CH, BE, AT, LU, DK**

1. A method for producing glucagon, comprising

   preparing a fusion protein shown by the following formula:

   X-Glu-Glucagon

   wherein X is a leader sequence consisting of amino acid sequence from Gln in the 4 position to Phe in the 95 position of human interferon-$\gamma$, Glu is a glutamic acid residue, and Glucagon represents the amino acid sequence of glucagon, by cultivating transformants obtained by introducing an expression vector for expressing said fusion protein into Escherichia Coli and allowing the Escherichia Coli to express said fusion protein; and treating said fusion protein with an enzyme that specifically hydrolyzes the peptide bonds at the carboxyl termini of the glutamic acid residues, resulting in cleavage of said fusion protein.

2. A method for producing glucagon, comprising

   preparing a fusion protein shown by the following formula:

   X-Glu-Glucagon                                                                                          (I)

   wherein X is a leader sequence consisting of amino acid sequence from Gln in the 4 position to Ser in the 135 position of human interferon-$\gamma$, Glu is a glutamic acid residue, and Glucagon represents the amino acid sequence of glucagon, by cultivating transformants obtained by introducing an expression vector for expressing said fusion protein into Escherichia coli and allowing the Escherichia coli to express said fusion protein; and

treating said fusion protein with an enzyme that specifically hydrolyzes the peptide bonds at the carboxyl termini of the glutamic acid residues, resulting in cleavage of said fusion protein.

3. A method according to claim 1 or 2, wherein said amino acid sequence of glucagon is shown by the following formula:

```
His Ser Gln Gly Thr Phe Thr Ser Asp Tyr
Ser Lys Tyr Leu Asp Ser Arg Arg Ala Gln
Asp Phe Val Gln Trp Leu Met Asn Thr        (II)
```

4. A method according to claim 1, 2 or 3 wherein said glucagon is recovered by the following procedure comprising,

(a) solubilizing said fusion protein with an appropriate denaturant or surfactant,

(b) lowering the concentration of said denaturant or surfactant,

(c) adding the enzyme to the mixture obtained in step (b), thereby obtaining a reaction mixture containing soluble intermediates arising from the partial decomposition of said fusion protein,

(d) desalinizing said reaction mixture to obtain a fraction containing said intermediates and said enzyme, and containing neither said denaturant nor said surfactant,

(e) allowing the reaction to further proceed by means of the enzyme contained in said fraction to release the glucagon, and

(f) isolating the glucagon released.

5. A method according to any preceding claim, wherein said enzyme is protease V8 derived from Staphylococcus aureus.

6. An expression vector comprising a DNA sequence encoding a fusion protein shown by the following formula:

X-Glu-Glucagon                                              (I)

wherein X is a leader sequence consisting of amino acid, sequence from Gln in the 4 position to Phe in the 95 position of human interferon-γ Glu is a glutamic acid residue, and Glucagon represents the amino acid sequence of glucagon.

7. An expression vector comprising a DNA sequence encoding a fusion protein shown by the following formula:

X-Glu-Glucagon                                              (I)

wherein X is a leader sequence consisting of amino acid sequence from Gln in the 4 position to Ser in the 135 position of human interferon-γ, Glu is a glutamic acid residue, and Glucagon represents the amino acid sequence of glucagon.

8. An expression vector according to claim 6 or 7, which is selected from the group consisting of: Trp pAT ΔCys huIFNγ/glucagon, Trp pAT ΔCys short huIFNγ/glucagon, and Trp pAT ΔCysII huIFNγ/glucagon.

9. A transformant obtained by introducing an expression vector of claim 6, 7 or 8 into a cell of Escherichia coli.

10. The method according to claim 1, wherein the Escherichia coli is a K12 strain.

11. The method according to claim 10, wherein said K12 strain is selected from the group consisting of C600, JM103 and AG-1.

**12.** The method according to claim 10, wherein said K12 strain is K12 C600.

**13.** The expression vector according to claim 6, 7 or 8 wherein said expression vector is expressed in Escherichia coli.

**14.** The expression vector according to claim 13, wherein said expression vector is expressed in Escherichia coli strain K12.

**15.** The expression vector according to claim 14, wherein said K12 strain is K12 C600.

**16.** A method for producing glucagon, comprising:

preparing a fusion protein by cultivating transformants obtained by introducing an expression vector for expressing a fusion protein shown by formula (I) into Escherichia coli and allowing the Escherichia coli to express the protein of formula (I):

$$\text{X-Glu-Glucagon} \tag{I}$$

wherein X is a leader sequence consisting of amino acid sequence from Gln in the 4 position to Phe in the 95 position of human interferon-$\gamma$, Glu is a glutamic acid residue, and Glucagon represents the amino acid sequence of glucagon, wherein said Glucagon is encoded by a DNA sequence shown by the following formula:

```
5'-   CACTCTCAGGGTACCTTCACTTCCGACTACTCTAAA
      TACCTGGACTCCCGTCGTGCTCAGGACTTCGTTCAG
      TGGCTGATGAACACC-3'; and
```

treating said fusion protein with an enzyme that specifically hydrolyzes the peptide bonds at the carboxyl termini of the glutamic acid residues, resulting in cleavage of said fusion protein.

**17.** A method for producing glucagon, comprising:

preparing a fusion protein by cultivating transformants obtained by introducing an expression vector for expressing a fusion protein shown by formula (I) into Escherichia coli and allowing the Escherichia coli to express the protein of formula (I) :

$$\text{X-Glu-Glucagon} \tag{I}$$

wherein X is a leader sequence consisting of amino acid sequence from Gln in the 4 position to Ser in the 135 position of human interferon-$\gamma$, Glu is a glutamic acid residue, and Glucagon represents the amino acid sequence of glucagon, wherein said Glucagon is encoded by a DNA sequence shown by the following formula:

```
5'-CACTCTCAGGGTACCTTCACTTCCGACTACTCTAAA
   TACCTGGACTCCCGTCGTGCTCAGGACTTCGTTCAG
   TGGCTGATGAACACC-3'                    (III)
```

treating said fusion protein with an enzyme that specifically hydrolyzes the peptide bonds at the carboxyl termini of the glutamic acid residues, resulting in cleavage of said fusion protein.

**Claims for the following Contracting States : ES, GR**

1. A method for producing glucagon, comprising

   preparing a fusion protein shown by the following formula:

   X-Glu-Glucagon                                                                                                   (I)

   wherein X is a leader sequence consisting of amino acid sequence from Gln in the 4 position to Phe in the 95 position of human interferon-γ, Glu is a glutamic acid residue, and Glucagon represents the amino acid sequence of glucagon, by cultivating transformants obtained by introducing an expression vector for expressing said fusion protein into Escherichia coli and allowing the Escherichia coli to express said fusion protein; and treating said fusion protein with an enzyme that specifically hydrolyzes the peptide bonds at the carboxyl termini of the glutamic acid residues, resulting in cleavage of said fusion protein.

2. A method for producing glucagon, comprising

   preparing a fusion protein shown by the following formula:

   X-Glu-Glucagon                                                                                                   (I)

   wherein X is a leader sequence consisting of amino acid sequence from Gln in the 4 position to Ser in the 135 position of human interferon-γ, Glu is a glutamic acid residue, and Glucagon represent the amino acid sequence of glucagon, by cultivating transformants obtained by introducing an expression vector for expressing said fusion protein into Escherichia coli and allowing the Escherichia coli to express said fusion protein; and treating said fusion protein with an enzyme that specifically hydrolyzes the peptide bonds at the carboxyl termini of the glutamic acid residues, resulting cleavage of said fusion protein.

3. A method according to claim 1 or 2, wherein said amino acid sequence of glucagon is shown by the following formula:

```
His Ser Gln Gly Thr Phe Thr Ser Asp Tyr
Ser Lys Try Leu Asp Ser Arg Arg Ala Gln
Asp Phe Val Gln Trp Leu Met Asn Thr          (II)
```

4. A method according to claim 1, 2 or 3, wherein said glucagon is recovered by the following procedure comprising,

   (a) solubilizing said fusion protein with an appropriate denaturant or surfactant,

   (b) lowering the concentration of said denaturant or surfactant,

   (c) adding the enzyme to the mixture obtained in step (b), thereby obtaining a reaction mixture containing soluble intermediates arising from the partial decomposition of said fusion protein,

   (d) desalinizing said reaction mixture to obtain a fraction containing said intermediates and said enzyme, and containing neither said denaturant nor said surfactant,

   (e) allowing the reaction to further proceed by means of the enzyme contained in said fraction to release the glucagon, and

   (f) isolating the glucagon released.

5. A method according to any preceding claim, wherein said enzyme is protease V8 derived from Staphylococcus aureus.

6. A method for producing an expression vector, wherein said expression vector is constructed using a DNA sequence encoding a fusion protein shown by the following formula:

X-Glu-Glucagon     (I)

wherein X is a leader sequence containing amino acid sequence from Gln in the 4 position to Phe in the 95 position of human interferon-γ, Glu is a glutamic acid residue, and Glucagon represents the amino acid sequence of glucagon.

7. A method for producing an expression vector, wherein said expression vector is constructed using a DNA sequence encoding a fusion protein shown by the following formula:

X-Glu-Glucagon     (I)

wherein X is a leader sequence containing amino acid sequence from Gln in the 4 position to Ser in the 135 position of human interferon-γ, Glu is a glutamic acid residue, and Glucagon represents the amino acid sequence of glucagon.

8. A method according to claim 6 or 7, wherein the expression vector is selected from the group consisting of: Trp pAT ΔCys hUIFNγ/glucagon, Trp pAT Δcys short huIFNγ/glucagon, and Trp pAT ΔCysII huIFNγ/glucagon.

9. A method for producing a transformant wherein an expression vector made by the method of claim 6, 7 or 8 is introduced into a cell of Escherichia coli.

10. The method according to claim 1, wherein the Escherichia coli is a K12 strain.

11. The method according to claim 10, wherein said K12 strain is selected from the group consisting of C600, JM103 and AG-1.

12. The method according to claim 10, wherein said K12 strain is K12 C600.

13. The method according to claim 6, 7 or 8, wherein said expression vector is expressed in Escherichia coli.

14. The method according to claim 13, wherein said expression vector is expressed in Escherichia coli strain K12.

15. The method according to claim 14, wherein said K12 strain is K12 C600.

16. A method for producing glucagon, comprising:

preparing a fusion protein by cultivating transformants obtained by introducing an expression vector for expressing a fusion protein shown by formula (I) into Escherichia coli and allowing the Escherichia coli to express the protein of formula (I):

X-Glu-Glucagon     (I)

wherein X is a leader sequence consisting of amino acid sequence from Gln in the 4 position to Phe in the 95 position of human interferon-γ, Glu is a glutamic acid residue, and Glucagon represents the amino acid sequence of glucagon, wherein said Glucagon is encoded by a DNA sequence shown by the following formula:

```
5' -   CACTCTCAGGGTACCTTCACTTCCGACTACTCTAAA
       TACCTGGACTCCCGTCGTGCTCAGGACTTCGTTCAG
       TGGCTGATGAACACC-3'; and
```

treating said fusion protein with an enzyme that specifically hydrolyzes the peptide bonds at the carboxyl termini of the glutamic acid residues, resulting in cleavage of said fusion protein.

**17.** A method for producing glucagon, comprising:

preparing a fusion protein by cultivating transformants obtained by introducing an expression vector for expressing a fusion protein shown by formula (I) into <u>Escherichia coli</u> and allowing the <u>Escherichia coli</u> to express the protein of formula (I) :

$$X\text{-Glu-Glucagon} \tag{I}$$

wherein X is a leader sequence consisting of amino acid sequence from Gln in the 4 position to Ser in the 135 position of human interferon-$\gamma$, Glu is a glutamic acid residue, and Glucagon represents the amino acid sequence of glucagon, wherein said Glucagon is encoded by a DNA sequence shown by the following formula:

```
5'-CACTCTCAGGGTACCTTCACTTCCGACTACTCTAAA
   TACCTGGACTCCCGTCGTGCTCAGGACTTCGTTCAG
   TGGCTGATGAACACC-3'                      (III)
```

treating said fusion protein with an enzyme that specifically hydrolyzes the peptide bonds at the carboxyl termini of the glutamic acid residues, resulting in cleavage of said fusion protein.


**Patentansprüche**


**Patentansprüche für folgende Vertragsstaaten : DE, GB, FR, IT, NL, SE, LI, CH, BE, AT, LU, DK**

**1.** Verfahren zur Herstellung von Glucagon, bei dem man ein Fusionsprotein gemäß der folgenden Formel:

$$X\text{-Glu-Glucagon}$$

herstellt, wobei X eine Leadersequenz ist, die aus der Aminosäuresequenz von Gln in Position 4 bis Phe in Position 95 von humanem Interferon-$\gamma$ besteht, wobei Glu ein Glutaminsäurerest ist und Glucagon die Aminosäuresequenz von Glucagon repräsentiert, indem man durch Einführung eines Expressionsvektors zur Expression des genannten Fusionsproteins in <u>Escherichia coli</u> erhaltene Transformanten kultiviert, und man <u>Escherichia coli</u> die Expression des Fusionsproteins erlaubt; und
man das Fusionsprotein mit einem Enzym behandelt, welches die Peptidbindungen an den Carboxytermini der Glutaminsäurereste spezifisch hydrolysiert, wodurch das Fusionsprotein gespalten wird.

**2.** Verfahren zur Herstellung von Glucagon, bei dem man ein Fusionsprotein gemäß der folgenden Formel:

$$X\text{-Glu-Glucagon} \tag{I}$$

herstellt, wobei X eine Leadersequenz ist, die aus der Aminosäuresequenz von Gln in Position 4 bis Ser in Position 135 von humanem Interferon-$\gamma$ besteht, wobei Glu ein Glutaminsäurerest ist und Glucagon die Aminosäuresequenz von Glucagon repräsentiert, indem man durch Einführung eines Expressionsvektors zur Expression des Fusions- proteins in <u>Escherichia coli</u> erhaltene Transformanten kultiviert, und man <u>Escherichia coli</u> die Expression des Fu- sionsproteins erlaubt; und

man das Fusionsprotein mit einem Enzym behandelt, welches die Peptidbindungen an den Carboxytermini der Glutaminsäurereste spezifisch hydrolysiert, wodurch das Fusionsprotein gespalten wird.

3. Verfahren nach Anspruch 1 oder 2, bei dem die Aminosäuresequenz von Glucagon durch die nachfolgende Formel wiedergegeben wird:

```
His Ser Gln Gly Thr Phe Thr Ser Asp Tyr
Ser Lys Tyr Leu Asp Ser Arg Arg Ala Gln
Asp Phe Val Gln Trp Leu Met Asn Thr            (II).
```

4. Verfahren nach Anspruch 1, 2 oder 3, bei dem das Glucagon durch das folgende Verfahren gewonnen wird, bei dem man

(a) das Fusionsprotein mit einem geeigneten Denaturierungsmittel oder Tensid solubilisiert,

(b) die Konzentration des Denaturierungsmittels oder Tensids verringert,

(c) der in Stufe (b) erhaltenen Mischung das Enzym zugibt, wodurch eine Reaktionsmischung erhalten wird, die lösliche Intermediate enthält, welche aus dem partiellen Abbau des Fusionsproteins entstanden sind,

(d) die Reaktionsmischung entsalzt, um eine Fraktion zu erhalten, welche die Intermediate und das Enzym, nicht aber das Denaturierungsmittel oder das Tensid enthält,

(e) die Reaktion durch das in der Fraktion enthaltene Enzym zur Freisetzung des Glucagons voranschreiten läßt, und man

(f) das freigesetzte Glucagon isoliert.

5. Verfahren nach irgendeinem vorhergehenden Anspruch, bei dem das Enzym die aus <u>Staphylococcus aureus</u> ab- geleitete Protease V8 ist.

6. Expressionsvektor umfassend eine DNA-Sequenz, die für ein Fusionsprotein gemäß der folgenden Formel kodiert:

X-Glu-Glucagon                                          (I),

wobei X eine Leadersequenz ist, die aus der Aminosäuresequenz von Gln in Position 4 bis Phe in Position 95 von humanem Interferon-γ besteht, wobei Glu ein Glutaminsäurerest ist und Glucagon die Aminosäuresequenz von Glucagon repräsentiert.

7. Expressionsvektor umfassend eine DNA-Sequenz, die für ein Fusionsprotein gemäß der folgenden Formel kodiert:

X-Glu-Glucagon                                          (I),

wobei X eine Leadersequenz ist, die aus der Aminosäuresequenz von Gln in Position 4 bis Ser in Position 135 von humanem Interferon-γ besteht, wobei Glu ein Glutaminsäurerest ist und Glucagon die Aminosäuresequenz von Glucagon respräsentiert.

8. Expressionsvektor nach Anspruch 6 oder 7, welcher ausgewählt ist aus der Gruppe bestehend aus: Trp pAT ∆Cys huIFNy/Glu-cagon, Trp pAT ∆Cys short huIFNγ/Glucagon und Trp pAT ∆CysII huIFNγ/Glucagon.

**9.** Transformante, erhalten durch Einführung eines Expressionsvektors gemäß Anspruch 6, 7 oder 8 in eine <u>Esche-richia coli</u>-Zelle.

**10.** Verfahren nach Anspruch 1, bei dem die <u>Escherichia coli</u>Zellen einem K12-Stamm angehören.

**11.** Verfahren nach Anspruch 10, bei dem der K12-Stamm ausgewählt wird aus der Gruppe bestehend aus C600, JM103 und AG-1.

**12.** Verfahren nach Anspruch 10, bei dem der K12-Stamm K12 C600 ist.

**13.** Expressionsvektor nach Anspruch 6, 7 oder 8, wobei der Expressionsvektor in <u>Escherichia coli</u> exprimiert wird.

**14.** Expressionsvektor nach Anspruch 13, wobei der Expressionsvektor im <u>Escherichia coli</u>-Stamm K12 exprimiert wird.

**15.** Expressionsvektor nach Anspruch 14, wobei der K12-Stamm K12 C600 ist.

**16.** Verfahren zur Herstellung von Glucagon, bei dem man:

ein Fusionsprotein herstellt, indem man durch Einführung eines Expressionsvektors zur Expression eines Fusionsproteins gemäß Formel (I) in <u>Escherichia coli</u> erhaltene Transformanten kultiviert, und man <u>Escherichia coli</u> die Expression des Proteins der Formel (I) erlaubt:

X-Glu-Glucagon (I),

wobei X eine Leadersequenz ist, die aus der Aminosäuresequenz von Gln in Position 4 bis Phe in Position 95 von humanem Interferon-γ besteht, wobei Glu ein Glutaminsäurerest und Glucagon die Aminosäuresequenz von Glucagon repräsentiert, wobei das Glucagon von einer DNA-Sequenz gemäß folgender Formel kodiert wird:

5′-CACTCTCAGGGTACCTTCACTTCCGACTACTCTAAA
TACCTGGACTCCCGTCGTGCTCAGGACTTCGTTCAG
TGGCTGATGAACACC-3′ ,

und man das Fusionsprotein mit einem Enzym behandelt, welches die Peptidbindungen an den Carboxytermini der Glutaminsäurereste spezifisch hydrolysiert, wodurch das Fusionsprotein gespalten wird.

**17.** Verfahren zur Herstellung von Glucagon, bei dem man:

ein Fusionsprotein herstellt, indem man durch Einführung eines Expressionsvektors zur Expression eines Fusionsproteins gemäß Formel (I) in <u>Escherichia coli</u> erhaltene Transformanten kultiviert, und man <u>Escherichia coli</u> die Expression des Proteins der Formel (I) erlaubt:

X-Glu-Glucagon (I),

wobei X eine Leadersequenz ist, die aus der Aminosäuresequenz von Gln in Position 4 bis Ser in Position 135 von humanem Interferon-γ besteht, wobei Glu ein Glutaminsäurerest ist und Glucagon die Aminosäuresequenz von Glucagon repräsentiert, wobei das Glucagon von einer DNA-Sequenz gemäß folgender Formel kodiert wird:

```
5'-CACTCTCAGGGTACCTTCACTTCCGACTACTCTAAA
   TACCTGGACTCCCGTCGTGCTCAGGACTTCGTTCAG
   TGGCTGATGAACACC-3'                          (III),
```

und man das Fusionsprotein mit einem Enzym behandelt, welches die Peptidbindungen an den Carboxytermini der Glutaminsäurereste spezifisch hydrolysiert, wodurch das Fusionsprotein gespalten wird.

**Patentansprüche für folgende Vertragsstaaten : ES, GR**

1. Verfahren zur Herstellung von Glucagon, bei dem man ein Fusionsprotein gemäß der folgenden Formel:

    X-Glu-Glucagon     (I)

    herstellt, wobei X eine Leadersequenz ist, die aus der Aminosäuresequenz von Gln in Position 4 bis Phe in Position 95 von humanem Interferon-γ besteht, wobei Glu ein Glutaminsäurerest ist und Glucagon die Aminosäuresequenz von Glucagon repräsentiert, indem man durch Einführung eines Expressionsvektors zur Expression des genannten Fusionsproteins in <u>Escherichia coli</u> erhaltene Transformanten kultiviert, und man <u>Escherichia coli</u> die Expression des Fusionsproteins erlaubt; und
    man das Fusionsprotein mit einem Enzym behandelt, welches die Peptidbindungen an den Carboxytermini der Glutaminsäurereste spezifisch hydrolysiert, wodurch das Fusionsprotein gespalten wird.

2. Verfahren zur Herstellung von Glucagon, bei dem man ein Fusionsprotein gemäß der folgenden Formel:

    X-Glu-Glucagon     (I)

    herstellt, wobei X eine Leadersequenz ist, die aus der Aminosäuresequenz von Gln in Position 4 bis Ser in Position 135 von humanem Interferon-γ besteht, wobei Glu ein Glutaminsäurerest ist und Glucagon die Aminosäuresequenz von Glucagon repräsentiert, indem man durch Einführung eines Expressionsvektors zur Expression des Fusionsproteins in <u>Escherichia coli</u> erhaltene Transformanten kultiviert, und man <u>Escherichia coli</u> die Expression des Fusionsproteins erlaubt; und
    man das Fusionsprotein mit einem Enzym behandelt, welches die Peptidbindungen an den Carboxytermini der Glutaminsäurereste spezifisch hydrolysiert, wodurch das Fusionsprotein gespalten wird.

3. Verfahren nach Anspruch 1 oder 2, bei dem die Aminosäuresequenz von Glucagon durch die nachfolgende Formel wiedergegeben wird:

```
His Ser Gln Gly Thr Phe Thr Ser Asp Tyr
Ser Lys Tyr Leu Asp Ser Arg Arg Ala Gln
Asp Phe Val Gln Trp Leu Met Asn Thr        (II).
```

4. Verfahren nach Anspruch 1, 2 oder 3, bei dem das Glucagon durch das folgende Verfahren gewonnen wird, bei dem man

    (a) das Fusionsprotein mit einem geeigneten Denaturierungsmittel oder Tensid solubilisiert,

    (b) die Konzentration des Denaturierungsmittels oder Tensids verringert,

    (c) der in Stufe (b) erhaltenen Mischung das Enzym zugibt, wodurch eine Reaktionsmischung erhalten wird, die lösliche Intermediate enthält, welche aus dem partiellen Abbau des Fusionsproteins entstanden sind,

(d) die Reaktionsmischung entsalzt, um eine Fraktion zu erhalten, welche die Intermediate und das Enzym, nicht aber das Denaturierungsmittel oder das Tensid enthält,

(e) die Reaktion durch das in der Fraktion enthaltene Enzym zur Freisetzung des Glucagons voranschreiten läßt, und man

(f) das freigesetzte Glucagon isoliert.

5. Verfahren nach irgendeinem vorhergehenden Anspruch, bei dem das Enzym die aus <u>Staphylococcus aureus</u> abgeleitete Protease V8 ist.

6. Verfahren zur Herstellung eines Expressionsvektors, bei dem der Expressionsvektor unter Verwendung einer DNA-Sequenz hergestellt wird, die für ein Fusionsprotein gemäß folgender Formel kodiert:

$$\text{X-Glu-Glucagon} \qquad (I),$$

wobei X eine Leadersequenz ist, welche die Aminosäuresequenz von Gln in Position 4 bis Phe in Position 95 von humanem Interferon-$\gamma$ enthält, wobei Glu ein Glutaminsäurerest ist und Glucagon die Aminosäuresequenz von Glucagon repräsentiert.

7. Verfahren zur Herstellung eines Expressionsvektors, bei dem der Expressionsvektor hergestellt wird unter Verwendung einer DNA-Sequenz, die für ein Fusionsprotein gemäß der folgenden Formel kodiert:

$$\text{X-Glu-Glucagon} \qquad (I),$$

wobei X eine Leadersequenz ist, welche die Aminosäuresequenz von Gln in Position 4 bis Ser in Position 135 von humanem Interferon-$\gamma$ enthält, wobei Glu ein Glutaminsäurerest ist und Glucagon die Aminosäuresequenz von Glucagon repräsentiert.

8. Verfahren nach Anspruch 6 oder 7, bei dem der Expressionsvektor ausgewählt wird aus der Gruppe bestehend aus: Trp pAT $\Delta$Cys huIFN$\gamma$/Glucagon, Trp pAT $\Delta$Cys short huIFN$\gamma$/Glucagon und Trp pAT $\Delta$CySII huIFN$\gamma$/Glucagon.

9. Verfahren zur Herstellung einer Transformante, bei dem ein Expressionsvektor, welcher gemäß dem Verfahren des Anspruchs 6, 7 oder 8 hergestellt wurde, in eine <u>Escherichia coli</u>-Zelle eingeführt wird.

10. Verfahren nach Anspruch 1, bei dem die <u>Escherichia coli</u>-Zellen einem K12-Stamm angehören.

11. Verfahren nach Anspruch 10, bei dem der K12-Stamm ausgewählt wird aus der Gruppe bestehend aus C600, JM103 und AG-1.

12. Verfahren nach Anspruch 10, bei dem der K12-Stamm K12 C600 ist.

13. Verfahren nach Anspruch 6, 7 oder 8, bei dem der Expressionsvektor in <u>Escherichia coli</u> exprimiert wird.

14. Verfahren nach Anspruch 13, bei dem der Expressionsvektor im <u>Escherichia coli</u>-Stamm K12 exprimiert wird.

15. Verfahren nach Anspruch 14, bei dem der K12-Stamm K12 C600 ist.

16. Verfahren zur Herstellung von Glucagon, bei dem man:

ein Fusionsprotein herstellt, indem man durch Einführung eines Expressionsvektors zur Expression eines Fusionsproteins gemäß Formel (I) in <u>Escherichia coli</u> erhaltene Transformanten kultiviert, und man <u>Escherichia coli</u> die Expression des Proteins der Formel (I) erlaubt:

$$\text{X-Glu-Glucagon} \qquad (I),$$

wobei X eine Leadersequenz ist, die aus der Aminosäuresequenz von Gln in Position 4 bis Phe in Position 95 von humanem Interferon-γ besteht, wobei Glu ein Glutaminsäurerest ist und Glucagon die Aminosäuresequenz von Glucagon repräsentiert, wobei das Glucagon von einer DNA-Sequenz gemäß folgender Formel kodiert wird:

```
5'-CACTCTCAGGGTACCTTCACTTCCGACTACTCTAAA
   TACCTGGACTCCCGTCGTGCTCAGGACTTCGTTCAG
   TGGCTGATGAACACC-3'    ,
```

und man das Fusionsprotein mit einem Enzym behandelt, welches die Peptidbindungen an den Carboxytermini der Glutaminsäurereste spezifisch hydrolysiert, wodurch das Fusionsprotein gespalten wird.

17. Verfahren zur Herstellung von Glucagon, bei dem man:

ein Fusionsprotein herstellt, indem man durch Einführung eines Expressionsvektors zur Expression eines Fusionsproteins gemäß Formel (I) in <u>Escherichia coli</u> erhaltene Transformanten kultiviert, und man <u>Escherichia coli</u> die Expression des Proteins der Formel (I) erlaubt:

$$X\text{-Glu-Glucagon} \qquad (I),$$

wobei X eine Leadersequenz ist, welche aus der Aminosäuresequenz von Gln in Position 4 bis Ser in Position 135 von humanem Interferon-γ besteht, wobei Glu ein Glutaminsäurerest ist und Glucagon die Aminosäuresequenz von Glucagon repräsentiert, wobei das Glucagon von einer DNA-Sequenz gemäß folgender Formel kodiert wird:

```
5'-CACTCTCAGGGTACCTTCACTTCCGACTACTCTAAA
   TACCTGGACTCCCGTCGTGCTCAGGACTTCGTTCAG
   TGGCTGATGAACACC-3'                          (III)
```

und man das Fusionsprotein mit einem Enzym behandelt, welches die Peptidbindungen an den Carboxytermini der Glutaminsäurereste spezifisch hydrolysiert, wodurch das Fusionsprotein gespalten wird.

## Revendications

**Revendications pour les Etats contractants suivants : DE, GB, FR, IT, NL, SE, LI, CH, BE, AT, LU, DK**

1. Procédé de production de glucagon, consistant

à préparer une protéine de fusion représentée par la formule suivante:

$$X\text{-Glu-Glucagon}$$

dans laquelle X est une séquence leader constituée d'une séquence d'aminoacides s'étendant de Gln dans la position 4 jusqu'à Phe dans la position 95 de l'interféron-γ humain, Glu est un résidu acide glutamique, et Glucagon représente la séquence d'aminoacides du glucagon, en cultivant des transformants obtenus en introduisant un vecteur d'expression pour exprimer ladite protéine de fusion dans <u>Escherichia Coli</u>, et en laissant <u>Escherichia. Coli</u> exprimer ladite protéine de fusion; et
à traiter ladite protéine de fusion avec une enzyme qui hydrolyse spécifiquement les liaisons peptidiques aux niveau des terminaisons carboxyle des résidus acide glutamique, de façon à obtenir le clivage de ladite protéine de fusion.

2. Procédé de production de glucagon, consistant

à préparer une protéine de fusion représentée par la formule suivante:

$$X\text{-Glu-Glucagon} \qquad (I)$$

dans laquelle X est une séquence leader constituée d'une séquence d'aminoacides s'étendant de Gln dans la position 4 jusqu'à Ser dans la position 135 de l'interféron-$\gamma$ humain, Glu est un résidu acide glutamique, et Glucagon représente la séquence d'aminoacides du glucagon, en cultivant les transformants obtenus en introduisant un vecteur d'expression pour exprimer ladite protéine de fusion dans Escherichia Coli, et en laissant Escherichia. Coli exprimer ladite protéine de fusion; et
à traiter ladite protéine de fusion avec une enzyme qui hydrolyse spécifiquement les liaisons peptidiques aux niveau des terminaisons carboxyle des résidus acide glutamique, de façon à obtenir le clivage de ladite protéine de fusion.

3. Procédé selon l'une des revendications 1 ou 2, dans lequel ladite séquence d'aminoacides du glucagon est représentée par la formule suivante:

```
His Ser Gln Gly Thr Phe Thr Ser Asp Tyr
Ser Lys Tyr Leu Asp Ser Arg Arg Ala Gln
Asp Phe Val Gln Trp Leu Met Asn Thr          (II)
```

4. Procédé selon l'une des revendications 1, 2 ou 3, dans lequel ledit glucagon est récupéré à l'aide du procédé suivant consistant:

(a) à solubiliser ladite protéine de fusion avec un dénaturant ou un tensioactif approprié,
(b) à abaisser la concentration dudit dénaturant ou tensioactif,
(c) à ajouter l'enzyme au mélange obtenu dans l'étape (b), de façon à obtenir un mélange réactionnel contenant des intermédiaires solubles provenant de la décomposition partielle de ladite protéine de fusion,
(d) à désaliniser ledit mélange de réaction pour obtenir une fraction contenant lesdits intermédiaires et ladite enzyme, et ne contenant ni ledit dénaturant ni ledit tensioactif,
(e) à laisser la réaction se poursuivre encore au moyen de l'enzyme contenue dans ladite fraction pour libérer le glucagon, et
(f) à isoler le glucagon libéré.

5. Procédé selon l'une des revendications précédentes, dans lequel ladite enzyme est la protéase V8 extraite de Staphylococcus aureus.

6. Vecteur d'expression comprenant une séquence d'ADN codant pour une protéine de fusion représentée par la formule suivante:

$$X\text{-Glu-Glucagon} \qquad (I)$$

dans laquelle X est une séquence leader constituée d'une séquence d'aminoacides s'étendant de Gln dans la position 4 jusqu'à Phe dans la position 95 de l'interféron-$\gamma$ humain, Glu est un résidu acide glutamique, et Glucagon représente la séquence des aminoacides du glucagon.

7. Vecteur d'expression comprenant une séquence d'ADN codant pour une protéine de fusion représentée par la formule suivante:

X-Glu-Glucagon                                                                                      (I)

dans laquelle X est une séquence leader constituée d'une séquence d'aminoacides s'étendant de Gln dans la position 4 jusqu'à Ser dans la position 135 de l'interféron-γ humain, Glu est un résidu acide glutamique, et Glucagon représente la séquence d'aminoacides du glucagon.

8. Vecteur d'expression selon l'une des revendications 6 ou 7, qui est choisi dans le groupe constitué par: Trp pAT Δcys huIFN γ/glucagon, Trp pAT ΔCys huIFN γ court/glucagon, et Trp pAT ΔCysII huIFN γ/glucagon.

9. Transformant obtenu en introduisant un vecteur d'expression de l'une des revendications 6, 7 ou 8 dans une cellule de Escherichia coli.

10. Procédé selon la revendication 1, dans lequel Escherichia coli est une souche K12.

11. Procédé selon la revendication 10, dans lequel ladite souche K12 est choisie dans le groupe formé par C600, JM103 et AG-1.

12. Procédé selon la revendication 10, dans lequel ladite souche K12 est K12 C600.

13. Vecteur d'expression selon l'une des revendications 6, 7 ou 8, ledit vecteur d'expression étant exprimé dans Escherichia coli.

14. Vecteur d'expression selon la revendication 13, ledit vecteur d'expression étant exprimé dans Escherichia coli souche K12.

15. Vecteur d'expression selon la revendication 14, dans lequel ladite souche K12 est la souche K12 C600.

16. Procédé de production de glucagon consistant:

à préparer une protéine de fusion par culture de transformants obtenus en introduisant un vecteur d'expression pour exprimer une protéine de fusion représentée par la formule (I) dans Escherichia coli et en laissant Escherichia coli exprimer la protéine de formule (I):

X-Glu-Glucagon                                                                                      (I)

dans laquelle X est une séquence leader constituée d'une séquence d'aminoacides s'étendant de Gln dans la position 4 jusqu'à Phe dans la position 95 de l'interféron-γ humain, Glu est un résidu acide glutamique, et Glucagon représente la séquence d'aminoacides du glucagon, dans laquelle ledit Glucagon est codé par une séquence d'ADN représentée par la formule suivante:

```
5'-CACTCTCAGGGTACCTTCACTTCCGACTACTCTAAA
   TACCTGGACTCCCGTCGTGCTCAGGACTTCGTTCAG
   TGGCTGATGAACACC-3'; et
```

à traiter ladite protéine de fusion avec une enzyme qui hydrolyse spécifiquement les liaisons peptidiques au niveau des terminaisons carboxyle des résidus acide glutamique de façon à obtenir le clivage de ladite protéine de fusion.

17. Procédé de production de glucagon consistant:

à préparer une protéine de fusion par culture de transformants obtenus en introduisant un vecteur d'expression pour exprimer une protéine de fusion représentée par la formule (I) dans Escherichia coli et en laissant Escherichia coli exprimer la protéine de formule (I):

X-Glu-Glucagon                                                                                                      (I)

dans laquelle X est une séquence leader constituée d'une séquence d'aminoacides s'étendant de Gln dans la position 4 jusqu'à Ser dans la position 95 de l'interféron-γ humain, Glu est un résidu acide glutamique, et Glucagon représente la séquence d'aminoacides du glucagon, dans laquelle ledit Glucagon est codé par une séquence d'ADN représentée par la formule suivante:

```
5'-CACTCTCAGGGTACCTTCACTTCCGACTACTCTAAA
    TACCTGGACTCCCGTCGTGCTCAGGACTTCGTTCAG
    TGGCTGATGAACACC-3';                              (III)
```

à traiter ladite protéine de fusion avec une enzyme qui hydrolyse spécifiquement les liaisons peptidiques au niveau des terminaisons carboxyle des résidus acide glutamique de façon à obtenir le clivage de ladite protéine de fusion.

**Revendications pour les Etats contractants suivants : ES, GR**

1. Procédé de production de glucagon, consistant

   à préparer une protéine de fusion représentée par la formule suivante:

   X-Glu-Glucagon

   dans laquelle X est une séquence leader constituée d'une séquence d'aminoacides s'étendant de Gln dans la position 4 jusqu'à Phe dans la position 95 de l'interféron-γ humain, Glu est un résidu acide glutamique, et Glucagon représente la séquence d'aminoacides du glucagon, en cultivant des transformants obtenus en introduisant un vecteur d'expression pour exprimer ladite protéine de fusion dans <u>Escherichia Coli</u>, et en laissant <u>Escherichia. Coli</u> exprimer ladite protéine de fusion; et
   à traiter ladite protéine de fusion avec une enzyme qui hydrolyse spécifiquement les liaisons peptidiques aux niveau des terminaisons carboxyle des résidus acide glutamique, de façon à obtenir le clivage de ladite protéine de fusion.

2. Procédé de production de glucagon, consistant

   à préparer une protéine de fusion représentée par la formule suivante:

   X-Glu-Glucagon                                                                                                 (I)

   dans laquelle X est une séquence leader constituée d'une séquence d'aminoacides s'étendant de Gln dans la position 4 jusqu'à Ser dans la position 135 de l'interféron-γ humain, Glu est un résidu acide glutamique, et Glucagon représente la séquence d'aminoacides du glucagon, en cultivant les transformants obtenus en introduisant un vecteur d'expression pour exprimer ladite protéine de fusion dans <u>Escherichia Coli</u>, et en laissant <u>Escherichia. Coli</u> exprimer ladite protéine de fusion; et
   à traiter ladite protéine de fusion avec une enzyme qui hydrolyse spécifiquement les liaisons peptidiques aux niveau des terminaisons carboxyle des résidus acide glutamique, de façon à obtenir le clivage de ladite protéine de fusion.

3. Procédé selon l'une des revendications 1 ou 2, dans lequel ladite séquence d'aminoacides du glucagon est représentée par la formule suivante:

```
His Ser Gln Gly Thr Phe Thr Ser Asp Tyr

Ser Lys Tyr Leu Asp Ser Arg Arg Ala Gln

Asp Phe Val Gln Trp Leu Met Asn Thr              (II)
```

**4.** Procédé selon l'une des revendications 1, 2 ou 3, dans lequel ledit glucagon est récupéré à l'aide du procédé suivant consistant:

(a) à solubiliser ladite protéine de fusion avec un dénaturant ou un tensioactif approprié,

(b) à abaisser la concentration dudit dénaturant ou tensioactif,

(c) à ajouter l'enzyme au mélange obtenu dans l'étape (b), de façon à obtenir un mélange réactionnel contenant des intermédiaires solubles provenant de la décomposition partielle de ladite protéine de fusion,

(d) à désaliniser ledit mélange de réaction pour obtenir une fraction contenant lesdits intermédiaires et ladite enzyme, et ne contenant ni ledit dénaturant ni ledit tensioactif,

(e) à laisser la réaction se poursuivre encore au moyen de l'enzyme contenue dans ladite fraction pour libérer le glucagon, et

(f) à isoler le glucagon libéré.

**5.** Procédé selon l'une des revendications précédentes, dans lequel ladite enzyme est la protéase V8 extraite de <u>Staphylococcus aureus</u>.

**6.** Procédé pour la production d'un vecteur d'expression dans lequel ledit vecteur d'expression est construit en utilisant une séquence d'ADN codant pour une protéine de fusion représentée par la formule suivante:

X-Glu-Glucagon                                    (I)

dans laquelle X est une séquence leader constituée d'une séquence d'aminoacides s'étendant de Gln dans la position 4 jusqu'à Phe dans la position 95 de l'interféron-$\gamma$ humain, Glu est un résidu acide glutamique, et Glucagon représente la séquence des aminoacides du glucagon.

**7.** Procédé pour la production d'un vecteur d'expression dans lequel ledit vecteur d'expression est construit en utilisant une séquence d'ADN codant pour une protéine de fusion représentée par la formule suivante:

X-Glu-Glucagon                                    (I)

dans laquelle X est une séquence leader constituée d'une séquence d'aminoacides s'étendant de Gln dans la position 4 jusqu'à Ser dans la position 135 de 1'interféron-$\gamma$ humain, Glu est un résidu acide glutamique, et Glucagon représente la séquence d'aminoacides du glucagon.

**8.** Procédé selon l'une des revendications 6 ou 7 dans lequel le vecteur d'expression est choisi dans le groupe formé par: Trp pAT $\Delta$cys huIFN $\gamma$/glucagon, Trp pAT $\Delta$cys huIFN $\gamma$ court/glucagon, et Trp pAT $\Delta$CySII huIFN $\gamma$/glucagon.

**9.** Procédé de production d'un transformant dans lequel un vecteur d'expression fabriqué par le procédé de l'une des revendications 6, 7 ou 8 est introduit dans une cellule de <u>Escherichia coli</u>.

**10.** Procédé selon la revendication 1, dans lequel <u>Escherichia coli</u> est une souche K12.

**11.** Procédé selon la revendication 10, dans lequel ladite souche K12 est choisie dans le groupe formé par C600, JM103 et AG-1.

**12.** Procédé selon la revendication 10, dans lequel ladite souche K12 est K12 C600.

**13.** Procédé selon l'une des revendications 6, 7 ou 8, dans lequel ledit vecteur d'expression est exprimé dans <u>Escherichia coli</u>.

**14.** Procédé selon la revendication 13, dans lequel ledit vecteur d'expression est exprimé dans Escherichia coli souche K12.

**15.** Procédé selon la revendication 14, dans lequel ladite souche K12 est la souche K12 C600.

**16.** Procédé de production de glucagon consistant:

à préparer une protéine de fusion par culture de transformants obtenus en introduisant un vecteur d'expression pour exprimer une protéine de fusion représentée par la formule (I) dans Escherichia coli et en laissant Escherichia coli exprimer la protéine de formule (I):

<div align="center">X-Glu-Glucagon (I)</div>

dans laquelle X est une séquence leader constituée d'une séquence d'aminoacides s'étendant de Gln dans la position 4 jusqu'à Phe dans la position 95 de l'interféron-$\gamma$ humain, Glu est un résidu acide glutamique, et Glucagon représente la séquence d'aminoacides du glucagon, dans laquelle ledit Glucagon est codé par une séquence d'ADN représentée par la formule suivante:

```
5'-CACTCTCAGGGTACCTTCACTTCCGACTACTCTAAA
   TACCTGGACTCCCGTCGTGCTCAGGACTTCGTTCAG
   TGGCTGATGAACACC-3'; et
```

à traiter ladite protéine de fusion avec une enzyme qui hydrolyse spécifiquement les liaisons peptidiques au niveau des terminaisons carboxyle des résidus acide glutamique de façon à obtenir le clivage de ladite protéine de fusion.

**17.** Procédé de production de glucagon consistant:

à préparer une protéine de fusion par culture de transformants obtenus en introduisant un vecteur d'expression pour exprimer une protéine de fusion représentée par la formule (I) dans Escherichia coli et en laissant Escherichia coli exprimer la protéine de formule (I):

<div align="center">X-Glu-Glucagon (I)</div>

dans laquelle X est une séquence leader constituée d'une séquence d'aminoacides s'étendant de Gln dans la position 4 jusqu'à Ser dans la position 95 de l'interféron-$\gamma$ humain, Glu est un résidu acide glutamique, et Glucagon représente la séquence d'aminoacides du glucagon, dans laquelle ledit Glucagon est codé par une séquence d'ADN représentée par la formule suivante:

```
5'-CACTCTCAGGGTACCTTCACTTCCGACTACTCTAAA
   TACCTGGACTCCCGTCGTGCTCAGGACTTCGTTCAG
   TGGCTGATGAACACC-3';                    (III)
```

à traiter ladite protéine de fusion avec une enzyme qui hydrolyse spécifiquement les liaisons peptidiques au niveau des terminaisons carboxyle des résidus acide glutamique de façon à obtenir le clivage de ladite protéine de fusion.

Fig. 1

Alu I    1               Kpn I              10

Met   His   Ser   Gln   Gly   Thr   Phe   Thr   Ser   Asp   Tyr   Ser   Lys

```
         ┌──────── Fr-1 ────────┐ ┌──── Fr-3 ────────┐ ┌──────── Fr-5 ────────┐
5' C T A T G C A C T C T C A G G G T A C C T T C A C T T C C G A C T A C T C T A A A T
3' G A T A C G T G A G A G T C C C A T G G A A G T G A A G G C T G A T G A
   └─── Fr-2 ───┘ └───── Fr-4 ──────┘         └─────── Fr-6 ───────┘
```

20

Tyr   Leu   Asp   Ser   Arg   Arg   Ala   Gln   Asp   Phe

```
       ┌──────── Fr-7 ────────┐ ┌──────── Fr-9 ────────┐
       A C C T G G A C T C C C G T C G T G C T C A G G A C T T
G A T T T A T G G A C C T G A G G G C A G C A C G A G T C C T G A A G C A A G T C A
└────── Fr-8 ──────┘ └────── Fr-10 ──────┘ └────── Fr-12 ──────┘
```

29         Hind III

Val   Gln   Trp   Leu   Met   Asn   Thr   Stop Stop

```
     ┌──────── Fr-11 ────────┐ ┌──────── Fr-13 ────────┐
     C G T T C A G T G G C T G A T G A A C A C C T A A T A A     3'
           C C G A C T A C T T G T G G A T T A T T T C G A   5'
           └──────── Fr-14 ────────┘ └──────── Fr-15 ────────┘
```

EP 0 381 433 B1

Fig. 2a

Fig. 2b

Fig. 2c

**Fig.3**

huIFN γ

133 134 135

Lys Arg Ser | Lys Ser Leu Val Asp Pro | Phe Arg | Cys Ser Glu

AAA AGG AGT | AAA TCT CTA GTC GAC CCG TTT CGC TGC AGC GAA

SalI    Kallikrein    PstI

Glucagon

His Ser Gln Gly Thr Phe --- Thr * *    (29)

CAC TCT CAG GGT ACC TTC    ACC TAA TAA AGCT | CTAGAG

KpnI

V8 protease cleavage site

Hind III Klenow    Xbal
Xbal Klenow

EP 0 381 433 B1

Fig. 4a

Fig. 4b

Fig. 5

Fig. 6

Fig. 7a

Sense primer →
5'-AGTTAACTAGTACGCAAGTT-3'

IFN α 80A2
MetCys

5'-TCGAACTAGTTAACTAGTACGCAAGTTCACGTAAAAAGGGTATCGATACTATGTGT ——————————
ClaI

| 136 | 137 | 138 | 139 | 140 | 141 | 142 | 143 | 144 | 145 | |
|-----|-----|-----|-----|-----|-----|-----|-----|-----|-----|-----|
| Lys | Tyr | Ser | Pro | Cys | Ala | Trp | Glu | Val | Val | Arg |
| AAA | TAC | AGC | CCT | TGT | GCC | TGT | GAG | GTT | GTC | AGA |

3' -ATG TCG GGA ACA CGG ACG TCC CAA C-5'

PstI

←——————— Antisense primer

138 139 140
Pro Cys Ala
CCT TGT GCC TGC A
PstI

Fig. 7b

Sense primer IX

Xho I        Glucagon ⟶

ΔCys huIFN γ→|        5'-CCGTTTCGCTCGAGCGAACAC-3'
LysArgSerLysSerLeuValAspProPheArgCysSerGluHisSerGln------
AAAAGGAGTAAATGTCTAGTCGACCCGTTTCGCTGCAGGAACACTCTCAG------
                                    Pst I

Glucagon ⟶|
----- MetAsnThrENDEND        Xba I
----- ATGAATACCTAATAAAGCTCTAGACGCT
        3'-GGATTATTTCGAGATCTGCGA-5'
                Antisense primer X

Fig. 7c

hu IFNγ——————|
LysArgSerLysSerLeuValAspProPheArgSerSerGluHisSer
AAAAGGAGTAAATCTCTAGTCGACCCGTTTCGCTCGAGCGAACACTCT
                   SalI          XhoI

Glucagon

Fig. 8

```
         10        20    .    30        40        50        60
TGTGATCTGCCTGAGACTCACAGCCTGGGTAATAGGAGGGCCTTGATACTCCTGGCACAA
C  D  L  P  Q  T  H  S  L  G  N  R  R  A  L  I  L  L  A  Q

         70        80        90        100       110       120
ATGGGAAGAATCTCTCATTTCTCCTGCCTGAAGGACAGATATGATTTCGGATTCCCCCAG
M  G  R  I  S  H  F  S  C  L  K  D  R  Y  D  F  G  F  P  Q

         130       140       150       160       170       180
GAGGTGTTTGATGGCAACCAGTTCCAGAAGGCTCAAGCCATCTCTGCCTTCCATGAGATG
E  V  F  D  G  N  Q  F  G  K  A  Q  A  I  S  A  F  H  E  M

         190       200       210       220       230       240
ATCCAGCAGACCTTCAATCTCTTCAGCACAAAGGATTCATCTGCTGCTTGGGATGAGACC
I  Q  Q  T  F  N  L  F  S  T  K  D  S  S  A  A  W  D  E  T

         250       260       270       280       290       300
CTCCTAGACAAATTCTACATTGAACTTTTCCAGCAACTGAATGACCTAGAAGCCTGTGTG
L  L  D  K  F  Y  I  E  L  F  Q  Q  L  N  D  L  E  A  C  V

         310       320       330       340       350       360
ACACAGGAGGTTGGGGTGGAAGAGATTGCCCTGATGAATGAGGACTCCATCCTGGCTGTG
T  Q  E  V  G  V  E  E  I  A  L  M  N  E  D  S  I  L  A  V

         370       380       390       400       410       420
AGGAAATACTTTCAAAGAATCACTCTTTATCTGATGGGGAAGAAATACAGCCCTTGTGCC
R  K  Y  F  Q  R  I  T  L  Y  L  M  G  K  K  Y  S  P  C  A

         430       440       450       460       470       480
TGGGAGGTTGTCAGAGCAGAAATCATGAGATCCTTCTCTTTTTCAACAAACTTGCAAAAA
W  E  V  V  R  A  E  I  M  R  S  F  S  F  S  T  N  L  Q  K

         490       500
GGATTAAGAAGGAAGGATTGA
G  L  R  R  K  D  *
```

Fig. 9a

Fig. 9b

Fig. 10a

Time (min.)

Fig. 10c

Time (min.)

50

Fig. 10b

Fig. 11

```
      His Ser Gln Gly Thr Phe Thr Ser Asp Tyr
5'-CAT TCA CAG GGC ACA TTC ACC AGT GAC TAC


      Ser Lys Tyr Leu Asp Ser Arg Arg Ala Gln
      AGC AAG TAT CTG GAC TCC AGG CGT GCC CAA


      Asp Phe Val Gln Trp Leu Met Asn Thr
      GAT TTT GTG CAG TGG TTG ATG AAT ACC-3'
```